# EUROPÄISCHE PATENTSCHRIFT

(11) **EP 1 448 179 B1**
(45) Veröffentlichungstag und Bekanntmachung des Hinweises auf die Patenterteilung: **09.08.2006**
(21) Anmeldenummer: 02790404.4
(22) Anmeldetag: 20.11.2002
(51) Int. Cl.: A61K 31/12, A61K 35/64, A61P 35/00

(54) **SUBSTITUIERTE BICYCLO 3.3.1 NONAN-2,4,9-TRIONE ALS PHARMAZEUTISCHE WIRKSTOFFE**
SUBSTITUTED BICYCLO 3.3.1 NONAN-2,4,9-TRIONES AS PHARMACEUTICAL ACTIVE INGREDIENTS
BICYCLO 3.3.1 NONAN-2,4,9-TRIONES SUBSTITUEES EN TANT QU'AGENTS PHARMACEUTIQUES

(30) Priorität: 21.11.2001 DE 10157033
(43) Veröffentlichungstag der Anmeldung: 25.08.2004
(73) Patentinhaber: Diagenics International Corporation, Woburn, MA 01801 (US)
(72) Erfinder: SEEBER, Siegfried, 45133 Essen (DE); HILGER, Ralf, Axel, 58256 Eneppetal (DE); DIAZ-CARBALLO, David, 45122 Essen (DE)
(74) Vertreter: Weiss, Peter
(86) Internationale Anmeldenummer: PCT/EP2002/012967
(87) Internationale Veröffentlichungsnummer: WO 2003/043622

(56) Entgegenhaltungen:
- DE OLIVEIRA CECILIA M A ET AL: "Floral resins of Clusia spp.: Chemical composition and biological function." TETRAHEDRON LETTERS, Bd. 37, Nr. 36, 1996, Seiten 6427-6430, XP002236050 ISSN: 0040-4039
- GUSTAFSON KIRK R ET AL: "The guttiferones, HIV-inhibitory benzophenones from Symphonia globulifera, Garcinia livingstonei, Garcinia ovalifolia and Clusia rosea." TETRAHEDRON, Bd. 48, Nr. 46, 1992, Seiten 10093-10102, XP001146562 ISSN: 0040-4020
- CUESTA-RUBIO OSMANY ET AL: "Polyisoprenylated benzophenones in Cuban propolis;biological activity of nemorosone." ZEITSCHRIFT FUER NATURFORSCHUNG SECTION C JOURNAL OF BIOSCIENCES, Bd. 57, Nr. 3-4, März 2002 (2002-03), Seiten 372-378, XP009008412 ISSN: 0939-5075
- DIAZ-CARBALLO DAVID ET AL: "Isolation and characterization of the novel antitumoral compound FB1, a potent telomerase- and topoisomerase-I-inhibitor." PROCEEDINGS OF THE AMERICAN ASSOCIATION FOR CANCER RESEARCH ANNUAL, Bd. 43, März 2002 (2002-03), Seite 250 XP001146555 93rd Annual Meeting of the American Association for Cancer Research;San Francisco, California, USA; April 06-10, 2002, March, 2002 ISSN: 0197-016X
- CHATURVEDULA V S P ET AL: "New cytotoxic coumarins and prenylated benzophenone derivatives from the bark of Ochrocarpos punctatus from the Madagascar rainforest" JOURNAL OF NATURAL PRODUCTS 2002 UNITED STATES, Bd. 65, Nr. 7, 2002, Seiten 965-972, XP002236051 ISSN: 0163-3864

## Beschreibung

Die vorliegende Erfindung betrifft die Verwendung von substituierten Bicyclo[3.3.1]nonan-2,4,9-trione.

Tumor- bzw. Krebserkrankungen sind kein einheitliches Krankheitsbild, sondern Oberbegriffe für eine Vielzahl verschiedener Formen bösartiger, maligner Erkrankungen. Nahezu jedes Gewebe unseres Körpers kann krebsige Entartungen hervorbringen, manchmal sogar mehrere unterschiedliche Typen. Jedes der Leiden wiederum hat seine eigenen Merkmale. Die Ursachen, die zu diesen Erkrankungen führen, sind oftmals sehr heterogen.

Trotz dieser Verschiedenartigkeit entstehen nahezu alle Tumore bzw. krebsige Entartungen durch sehr ähnliche, grundlegende molekulare bzw. zelluläre Prozesse, In den letzten zwei Jahrzehnten hat die Forschumg erstaunliche Erkenntnisfortschritte erhracht, was die fundamentalsten Prozesse des Krebs- bzw. Tumorgeschehens auf molekularer Ebene anbelangt.

Träger der Erbinformationen sind die DNA-Moleküle der Chromosomen im Zellkern. Zwei Klassen von Genen, die zusammen nur einen kleinen Anteil der gesamten Ausstattung einer Zelle ausmachen, spielen für die Krebsentstehung eine wesentliche Rolle, nämlich insbesondere Proto-Onkogene (Krebsgen-Vorläufer) und Tumorsuppressor-Gene (tumorunterdrückende Gene). In ihrer normalen Form dirigieren sie den Lebenszylklus der Zelle, sie steuern die verwickelte Abfolge von Vorgängen, durch die sich eine Zelle vergrößert und bei Bedarf teilt. Während Proto-Onkogene das Zellwachstum fördern, wird es durch Tumorsuppressor-Gene gebremst. Zusammen sind diese beiden Klassen von Genen für einen Großteil der unkontrollierten Zellvermehrungsprozesse in menschlichen Tumoren verantwortlich: Wenn z. B. ein Proto-Onkogen in der Regulator- oder in der Strukturregion mutiert, kann es passieren, daß nun zuviel von seinem wachstumsfördernden Protein hergestellt wird oder daß dieses nun übermäßig aktiv ist; aus dem Proto-Onkogen ist dann ein krebsbegünstigendes Onkogen geworden, das die Zellen zu übermäßiger Vermehrung anregt. Demgegenüber tragen Tumorsuppressor-Gene zur Krebsentstehung bei, wenn sie durch Mutationen inaktiviert werden; als Folge verliert die Zelle funktionsfähige Suppressor-Proteine und damit entscheidende Wachstumsbremsen, die sie normalerweise an einer unangemessenen Vermehrung hindern.

In normalen Körperzellen ist ein Notmechanismus gegen unbegrenzte Vermehrung eingebaut, es handelt sich dabei um eine Art Zählwerk, das jede Zellteilung registriert und nach einer bestimmten Anzahl von Generationen Einhalt gebietet. Nach einer bestimmten, in etwa voraussagbaren Zahl von Zellteilungen bzw. Verdoppelungen hört das Wachstum normaler Zellen auf. Dieser Prozeß wird als Zellalterung oder Seneszenz bezeichnet.

Für diesen Prozeß der Zellalterung oder Senenszenz auf molekularer Ebene verantwortlich sind die DNA-Segmente an den Enden der Chromosomen, die sogenannten Telomere. Sie registrieren sozusagen, wieviele Vermehrungszyklen eine Zellpopulation durchläuft und leiten ab einem bestimmten Zeitpunkt die Seneszenz bzw. die Krise ein. Dadurch begrenzen Sie die Fähigkeit einer Zellpopulation, uneingeschränkt zu wachsen. In den meisten, gesunden menschlichen Zellen verkürzen sich die Telomeren bei jeder Zellteilung um ein kleines Stück, wenn die Chromosomen repliziert werden. Sind sie auf eine bestimmte kritische Länge geschrumpft, ist das für die Zelle das Signal, in das Seneszenzstadium einzutreten; ignoriert die Zelle die Warnung, verkürzen sich die Telomere weiter, bis es schließlich zur Krise kommt: Bei extrem kurzen Telomeren verknüpfen sich Chromosomen miteinander oder zerbrechen, das angerichtete genetische Chaos ist für die Zelle tödlich.

Der Alterungs- bzw. Seneszenzprozeß in normalen Zellen, d. h. der Verlust des Teilungsvermögens, dem normale Zellen unterliegen, ist also dadurch bedingt, daß Chromosomen in normalen sterblichen Zellen etwa 50 bis 200 DNA-Nucleotide pro Zellteilung an ihren telomeren Enden verlieren. Der Verlust dieser endständigen Nukleotide (Telomere) hat die Funktion einer Art mitotischer Uhr, welche die Anzahl der Zellteilungen registriert. Der Erhalt der Telomeren scheint für Zellen erforderlich zu sein, um dem Seneszenzprozeß zu entkommen und sich undefiniert teilen zu können.

Der zuvor beschriebene Schutzmechanismus ist im Zuge der Entartung bei den meisten Krebs- bzw. Tumorzellen außer Kraft gesetzt, und zwar durch die Aktivierung eines Gens, das die Synthesevorschrift für ein Enzym namens Telomerase enthält. Dieses Enzym vervollständigt systematisch die sich sonst verkürzenden Telomerabschnitte, erhält sie dadurch auf Dauer und ermöglicht der Zelle eine quasi unbegrenzte Weiterteilung. Bei den meisten Tumor- bzw. Krebserkrankungen resultiert also die Unsterblichkeit der Zellen aus der Beibehaltung kurzer, aber stabiler Telomere aufgrund der Einwirkung von Telomerase. Die resultierende potentielle Unsterblichkeit der Zellen ist in mehrfacher Hinsicht ungünstig: Zum einen trägt sie dazu bei, daß ein Tumor sehr groß werden kann, und zum anderen gibt sie präkanzerösen oder bereits echten Krebszellen Zeit, weitere Mutationen anzuhäufen, die ihre Fähigkeiten steigern, sich zu vermehren, in andere Gewebe einzudringen und schließlich zu metastasieren.

Man hat herausgefunden, daß das unbegrenzte Zellwachstum entarteter Zellen in circa 80 % aller Tumorerkrankungen auf einer gestörten Regulation der Telomerase basiert. Die Behandlung von Tumor- bzw. Krebserkrankungen über Telomeraseinhibitoren scheint daher eine vielversprechende Therapie für diese Erkrankungen zu ermöglichen. Folglich wurde im Stand der Technik bereits vielfach der Versuch unternommen, Wirkstoffe zur Inhibierung von Telomerase zu entwickeln.

So wird beispielsweise in der WO 00/74667 A2 die Verwendung von Telomeraseinhibitoren (z. B. AZT) in Kombination mit einem die Telomerenzerstörung induzierenden Wirkstoff (z. B. Paclitaxel) zur Krebsbehandlung vorgeschlagen. Die WO 99/65875 A1 und die US-A-5 863 936 schlagen die Verwendung spezieller heterobicyclischer Systeme als Telomerascinhibitoren zur Therapie von Krebserkrankungen vor. Catechinderivate, wie sie in der europäischen Offenlegungsschrift EP 0 938 897 A1 beschreiben sind, sollen ebenfalls als Telomeraseinhibitoren zur Krebsbehandlung eingesetzt werden können

Des weiteren wird gemäß dem Stand der Technik versucht, über andere Mechanismen auf molekularer bzw. zellulärer Ebene in die Vermehrung von Tumor- bzw. Krebszellen einzugreifen: So werden zur Behandlung von Tumor- bzw. Krebserkrankungen gemäß dem Stand der Technik vielfach Alkylierungsreagenzien eingesetzt, die über eine Alkylierung der DANN letztendlich eine direkte Schädigung der DANN der Tumor- bzw. Krebszellen induzierten (z.B. Cyclophosphamid, Ifosfamid, Carmustin, Chlorambucil etc.). Gleichermaßen kommen für die Tumor- bzw. Krebstherapie Substanzen zum Einsatz, die über die Inhibierung von Topoisomerasen in die Replikation der . Tumor- bzw. Krebszellen eingreifen (z.B. Camptoceticin, 9-Aminocamptothecin, Irinotecan, Topotecan, Doxorubicin etc.) (vgl. beispielsweise Clive Page et.al., Integrated Pharmacology, Mosby, 1997).

Während es - neben alkylierenden Wirkstoffen - einige Topoisomerasenihibitoren in der Klinik gibt, gibt es zur Zeit in der Klinik keinen Wirkstoff zur Behandlung diverser Tumore, welcher über die Inhibition der Telomerase wirkt oder gar die Eigenschaften einer Inhibition der Topoisomerase und der Telomerase vereint.

Aus DE OLIVEIRA CECILIA M A ET AL: "Floral resins of Clusia spp.: Chemical composition and biological function." TETRAHEDRON LETTERS, Bd. 37, Nr. 36, 1996, Seiten 6427-6430, XP002236050 ISSN: 0040-4039" sind Nonantrione (Benzophenone aus Clusia ssp.) bekannt. Es wird erwähnt, dass einige dieser Stoffe Aktivitäten als HIV-Inhibitoren zeigen. D.h., es wird die Verwendung als Virostatika in Erwägung gezogen.

GUSTAFSON KIRK R ET AL: "The guttiferones, HIV-Inhibitory benzophenones from Symphonia globulifera, Garcinia livingstonei, Garcinia olvalifolia and Clusia rosea." TETRAHEDRON, Bd. 48, Nr. 46, 1992, Seiten 10093-10102, XP001146562 ISSN: 0040-4020 offenbart die antivirale Wirksamkeit von Benzophenon-substituierten (3.3.1) Nonantrionen. Es wird ebenfalls auf die inhibitorische Aktivität dieser Nonantrionen auf HI-Viren hingewiesen.

Die Aufgabe der vorliegenden Erfindung besteht darin, eine weitere Verwendung für die oben erwähnten substituierten Bicyclo [3.3.1] nonan-2,4,9-trione zu finden.

Der Anmelder hat nun überraschenderweise herausgefunden, daß substituierte Bicyclo[3.3.1]nonan-2,4,9-trione der allgemeinen Formel (I) 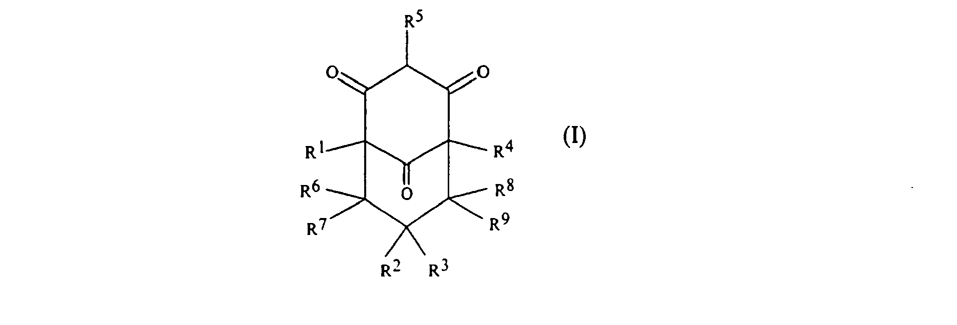 in der:
- R¹, R², R⁴ und R⁵, gleich oder verschieden, unabhängig voneinander eine der folgenden Gruppen darstellen: 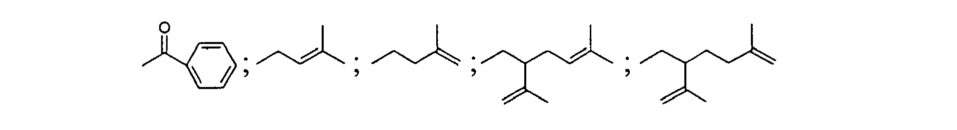 jedoch mit der Maßgabe, daß, wenn einer der Reste R¹ oder R² oder R⁴ oder R⁵ eine Benzoylgruppe darstellt, keiner der anderen dieser Reste eine Benzoylgruppe darstellt;
- R³ ein Wasserstoffatom darstellt;
- R⁶ und R⁷ beide jeweils eine Methylgruppe oder aber jeweils ein Wasserstoffatom darstellen und R⁸ und R⁹ beide jeweils eine Methylgruppe oder aber jeweils ein Wasserstoffatom darstellen, jedoch mit der Maßgabe, daß, wenn R⁶ und R⁷ Methylgruppen darstellen, R⁸ und R⁹ Wasserstoffatome sind und daß, wenn R⁸ und R⁹ Methylgruppen darstellen, R⁶ und R⁷ Wasserstoffatome sind;
und ihre physiologisch verträglichen Salze, Hydrate, Isomere, insbesondere Stereoisomere, Tautomere und Konstitutionsisomere, Derivate, sich insbesondere für die prophylaktische und/oder therapeutische (kurative) Behandlung von Tumor- bzw. Krebserkrankungen eignen.

Ein Gegenstand der vorliegenden Erfindung ist somit die Verwendung der zuvor genannten Verbindungen der allgemeinen Formel (I) einschließlich ihrer physiologisch verträglichen Salze, Hydrate, Isomeren, insbesondere Stereoisomeren, Tautomeren und Konstitutionsisomeren, Derivate, Prodrugs und Metabolite zur Herstellung von Arzneimitteln zur prophylaktischen und/oder therapeutischen Behandlung von Tumor- und/oder Krebserkrankungen, und zwar von Menschen wie von Tieren, d. h. sowohl im Bereich der Human- als auch der Veterinärmedizin.

Physiologisch verträgliche bzw. unbedenkliche Salze der Verbindungen der allgemeinen Formel (I) können beispielsweise Salze mit Mineralsäuren, Carbonsäuren oder Sulfonsäuren sein; besonders bevorzugt sind z. B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure. Als physiologisch verträgliche bzw. unbedenkliche Salze können aber beispielsweise auch Salze mit üblichen Basen genannt werden, wie beispielsweise Alkalimetallsalze (z. B. Natrium- oder Kaliumsalze), Erdalkalisalze (z. B. Calcium- oder Magnesiumsalze) oder Ammoniumsalze, abgeleitet von Ammoniak oder organischen Aminen wie beispielsweise Diethylamin, Triethylamin, Ethyldiisopropylamin, Procain, Dibenzylamin, N-Methylmorpholin, Dihydroabiethylamin, 1-Ephenamin oder Methylpiperidin.

Die vorliegende Erfindung umfaßt auch die Hydrate der Verbindungen der allgemeinen Formel (I). Als Hydrate werden erfindungsgemäß solche Formen der Verbindungen der obigen allgemeinen Formel (I) bezeichnet, welche in festem oder flüssigem Zustand durch Hydratation mit Wasser eine Molekülverbindung (Hydrat) bilden. In den Hydraten sind die Wassermoleküle durch zwischenmolekulare Kräfte, insbesondere Wasserstoff-Brückenbindungen, angelagert. Feste Hydrate enthalten Wasser als sogenanntes Kristallwasser in stöchiometrischen oder nichtstöchiometrischen Verhältnissen, wobei die Wassermoleküle hinsichtlich ihres Bindungszustands nicht gleichwertig sein müssen. Beispiele für Hydrate sind Sesquihydrate, Monohydrate, Dihydrate, Trihydrate etc. Gleichermaßen kommen erfindungsgemäß auch die Hydrate von Salzen der Verbindungen der allgemeinen Formel (I) in Betracht.

Die vorliegende Erfindung umfaßt auch die Isomere der Verbindungen der allgemeinen Formel (I). Der Begriff der Isomere im Sinne der vorliegenden Erfindung wird als umfassende Bezeichnung aller möglichen Isomerieformen verwendet. Nichtbeschränkende Beispiele für Isomere, die von der vorliegenden Erfindung mitumfaßt sind, sind insbesondere Stereoisomere, Tautomere und Konstitutionsisomere.

So können die Verbindungen der allgemeinen Formel (I) in Abhängigkeit von dem Substitutionsmuster in isomeren Formen vorkommen, insbesondere als Stereoisomere, z. B. entweder als sich wie Bild und Spiegelbild verhaltende Stereoisomere (Enantiomere) oder aber als sich nicht wie Bild und Spiegelbild verhaltende Stereosisomere (Diastereomere). Die vorliegende Erfindung umfaßt alle stereoisomeren Formen der Verbindungen der allgemeinen Formel (I), d. h. sowohl die Enantiomeren als auch die Diastereomeren wie auch deren jeweilige Mischungen. Die enantiomeren Formen lassen sich ebenso wie die Diastereomeren in an sich bekannter Weise in stereoisomer einheitliche Bestandteile trennen.

Weiterhin können die Verbindungen der allgemeinen Formel (I) in tautomeren Formen vorliegen. Dies ist dem Fachmann bekannt, und derartige Verbindungen sind ebenfalls vom Umfang der vorliegenden Erfindung mitumfaßt. Beispiele für tautomere Formen sind beispielsweise die folgenden Keto-Enol-Tautomere:

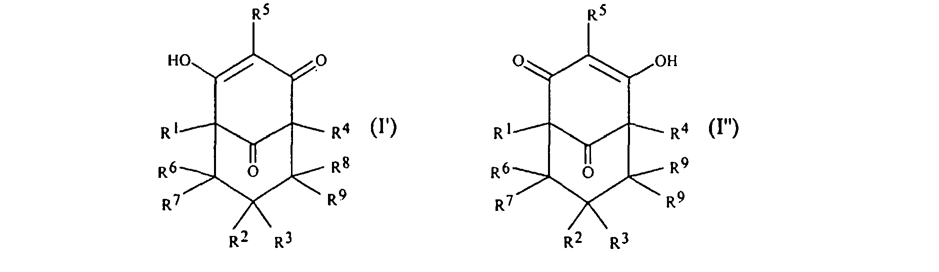

Wenn R⁵ beispielsweise eine Benzoylgruppe darstellt, ist eine weitere KetoEnol-Tautomerieform möglich.

Des weiteren können die Verbindungen der allgemeinen Formel (I) als Konstitutionsisomere, insbesondere Stellungsisomere (Substitutionsisomere), vorliegen. Dies ist dem Fachmann bekannt, und derartige Verbindungen sind ebenfalls vom Umfang der vorliegenden Erfindung umfaßt.

Die vorliegende Erfindung umfaßt auch Derivate der Verbindungen der allgemeinen Formel (I) (so z. B. Ether wie Alkylether, wie z. B. Methylether, die beispielsweise durch Alkylierung der tautomeren Formen (I') bzw. (I") erhalten werden können). Erfindungsgemäß besonders bevorzugte Derivate der Verbindungen der allgemeinen Formel (I) sind die pegylierten Derivate, insbesondere die Polyalkylenglykolether, vorzugsweise Polyethylenglykolether (PEG-Ether), der tautomeren Formen (I') und (I"). Des weiteren sind auch chemische Modifikationen möglich, insbesondere in bezug auf die Substituenten R¹ bis R⁹ (z. B. durch Seitengruppenderivatisierung).

Gleichermaßen umfaßt die vorliegende Erfindung auch Prodrugs und Metabolite der Verbindungen der allgemeinen Formel (I). Als Prodrugs werden erfindungsgemäß insbesondere solche Formen der Verbindungen der obigen allgemeinen Formel (I) bezeichnet, welche selbst biologisch aktiv oder inaktiv sein können, jedoch in die entsprechende biologisch aktive Form überführt werden können (beispielsweise metabolitisch, solvolytisch oder auf andere Weise). Als Metabolite werden erfindungsgemäß insbesondere die aus dem Stoffwechsel resultierenden oder die im Stoffwechsel umgesetzten Produkte der Verbindungen der allgemeinen Formel (I) bezeichnet.

Die Verbindungen der allgemeinen Formel (I) sind Naturstoffe und sind beispielsweise erhältlich durch chromatographische Abtrennung aus dem Propolisextrakt karibischer Bienen. Propolis ist eine dunkelgelbliche bis hellbraune, harzartige, zwischen den Fingern erweichende Masse mit würzig-balsamischem Geruch, die von Bienen insbesondere von den Knospen von Blüten gesammelt und im Bienenstock als Überzug der Wände und zum Befestigen der Waben benutzt wird. Propolis ist chemisch sehr komplex aufgebaut und seine genaue Zusammensetzung ist von der jeweiligen lokalen Flora abhängig. Propolis enthält mehr als zweihundert verschiedene chemische Substanzen, hierunter größere Anteile Wachse und Harze, etherische und aromatische Öle sowie eine Vielzahl anderer chemischer Substanzen, hierunter beispielsweise Flavonoide, Kaffeesäurederivate und im Fall des karibischen Propolis auch die Verbindungen der allgemeinen Formel (I).

Des weiteren können die Verbindungen der allgemeinen Formel (I) auch direkt aus dem Pflanzensaft bzw. -harz (z. B. aus dem Harz, dem Latex, den Blättern, der Blüten etc.) verschiedener *Clusia*-Arten, Familie der Clusiaceae oder Guttiferae, isoliert werden (z. B. aus *Clusia grandiflora, Clusia rosea* oder *Clusia renggerioides):*

So beschreiben A. J. Marsaioli et al. "The Ecosystem of Microorganisms, Bees, and *Clusia* Floral Resin and Oils, from the Chemistry Point of View", Pure Appl. Chem., Band 70, 11, Seiten 2116 ff. (1998), C. M. A. de Oliveira et al. "Two polyisoprenylated benzophenones from the floral resins of three *Clusia* species", Phytochemistry 50 (1999), Seiten 1073-1079 und A. L. M. Porto et al. "Polyisopenylated benzophenones from *Clusia* floral resins", Phytochemistry 55 (2000), Seiten 755-768, die Isolierung verschiedener Clusianonderivate der obigen allgemeinen Formel (I) ausgehend von den Harzen bzw. Inhaltsstoffen verschiedener Pflanzen der Art *Clusia* (Familie der Clusiaceae oder Guttiferae) durch chromatographische Methoden (Umsetzung der Harze mit Diazomethan in Diethylether und anschließende Säulenchromatographie an einer mit Silicagel gepackten Säule mit Hexan/Ethylacetat oder Hexan/Diethylether als Eluiermittel (Gradientensäule), gefolgt von einer präparativen Dünnschichtchromatographie auf Silicagel/Silbernitrat mit Benzol/Ethylacetat als Eluiermittel).

Die Isolierung des Clusianons selbst ausgehend von dem Harz von Pflanzen der Familie der Clusiaceae oder Guttiferae, nämlich *Clusia congestiflora*, und dessen Strukturaufklärung ist von L. E. McCandlish et al. "The Structures of Two Derivatives of Bicyclo[3.3.1]nonane-2,4,9-trione. A Natural Product: Clusianone, C₃₃H₄₂O₄, and Trimethylated Catechinic Acid, C₁₈H₂₀O₆" in Acta Cryst. (1976). B32, Seiten 1793-1801 beschrieben.

M. H. Santos et al. "Efeito de constituintes quimicos extraidos do fruto de *Rheedia gardneriana* (bacupari) sobre bactérias patogênicas" in Brazilian Journal of Pharmaceutical Sciences (Revista Brasileira de Ciências Farmaceuticas), Band 35, Nr. 2, Juli/Dezember 1999, Seiten 297-301 und "Epiclusianon: A New Natural Product Derivative of Bicyclo[3.3.1]nonane-2,4,9-trione", Acta Cryst. (1998), C54, Seiten 1990-1992, beschreiben die Isolierung von Epiclusianon aus dem Blütenextrakt von *Rheedia gardneriana* (bacuparí), einer Pflanze der Familie der Clusiaceae.

F. delle Monache et al. "Prenylated Benzophenones From *Clusia Sandiensis*", Phytochemistry, Band 30, Nr. 6, Seiten 2003-2005 (1991) beschreiben die Isolierung verschiedener Clusianonderivate ausgehend von dem Pflanzenharz von *Clusia Sandiensis* mittels chromatographischer Methoden (Säulenchromatographie, gefolgt von präparativer Dünnschichtchromatographie).

Der gesamte Offenbarungsgehalt der zuvor genannten Dokumente des Standes der Technik in bezug auf die Herstellung bzw. Isolierung der Verbindungen der allgemeinen Formel (I) wird hiermit durch Bezugnahme eingeschlossen.

Der Anmelder hat nun völlig überraschend herausgefunden, daß die Verbindungen der allgemeinen Formel (I) einschließlich ihrer physiologisch verträglichen Salze, Hydrate, Isomeren, insbesondere Stereoisomeren, Tautomeren und Konstitutionsisomeren, Derivate, Prodrugs und Metabolite ein nicht vorhersehbares, wertvolles pharmakologisches Wirkspektrum zeigen und sich daher zur Prophylaxe und/oder Behandlungen von Erkrankungen, insbesondere Krebs- bzw. Tumorerkrankungen aller Art, aber auch von Viruserkrankungen aller Art, eignen.

Die Verbindungen der allgemeinen Formel (I) einschließlich ihrer physiologisch verträglichen Salze, Hydrate, Isomeren, insbesondere Stereoisomeren, Tautomeren und Konstitutionsisomeren, Derivate, Prodrugs und Metabolite können folglich bevorzugt eingesetzt werden in Arzneimitteln zur Prophylaxe und/oder Behandlung von Erkrankungen, vorzugsweise von Tumor- und Krebserkrankungen (Cytostatika) und von Viruserkrankungen (Antivirusmittel bzw. Virostatika). Weiterer Gegenstand der vorliegenden Erfindung sind somit auch pharmazeutische Zusammensetzungen oder Arzneimittel, insbesondere Cytostatika oder Antivirusmittel (Virostatika), die mindestens eine Verbindung der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite, vorzugsweise in therapeutisch wirksamen Mengen, zusammen mit einem pharmazeutisch verträglichen, im wesentlichen nichttoxischen Träger oder Exzipienten enthalten.

Der Anmelder hat überraschend herausgefunden, daß die Verbindungen der allgemeinen Formel (I) unter anderem als Topoisomeraseinhibitoren, insbesondere als Inhibitoren von Topoisomerase I, wirken. Des weiteren zeigen sie im allgemeinen auch eine telomeraseinhibitorische Wirkung. Letztendlich induzieren die erfindungsgemäß eingesetzten Verbindungen der allgemeinen Formel (I) die Zerstörung der DNA in Tumor- bzw. Krebszellen.

Die topoisomeraseinhibitorische und telomeraseinhibitorische Wirkung der erfindungsgemäß verwendeten Verbindungen der allgemeinen Formel (I) ist deutlich höher als bei den aus dem Stand der Technik bekannten Verbindungen. So zeigen beispielsweise die Verbindungen der allgemeinen Formel (I) in in-vitro-Versuchen eine erheblich stärkere Inhibition von Topoisomerase I als herkömmliche Therapeutika (z. B. Topotecan). Zusätzlich wirken die Verbindungen der allgemeinen Formel (I) auch als Regulatoren innerhalb der MAP-Kinase-Signaltransduktion.

Die Tatsache, daß die Verbindungen der allgemeinen Formel (I) die Eigenschaften der Inhibition von Topoisomerase und Telomerase vereinen, erklärt ihre wertvollen Eigenschaften und ihre besondere Eignung in bezug auf ihre Verwendung zur prophylaktischen und therapeutischen (kurativen) Behandlung von Tumor- und Krebserkrankungen aller Art (Primärtumore, Metastasen, Präkanzerosen bzw. Krebsvorstufen, benigne wie maligne Tumore etc.). Aus dem Stand der Technik dagegen ist kein Wirkstoff bzw. kein Präparat bekannt, welches die Eigenschaften der Inhibition von Topoisomerase einerseits und Telomerase andererseits in einem einzigen Molekül vereint.

Die erfindungsgemäß verwendeten Wirkstoffe der allgemeinen Formel (I) zeigen nicht nur antitumorale Wirkung, sondern zusätzlich auch antimetastatische Wirkung.

Die erfindungsgemäß verwendeten Wirkstoffe der allgemeinen Formel (I) zeigen überraschend auch gegenüber Karzinomen, welche gegen etablierte Chemotherapeutika definierte Resistenzen aufweisen, eine unerwartet gute cytostatische Wirkung. Weiter wurden keine Resistenzen gegen die Wirkstoffe der allgemeinen Formel (I) beobachtet. Des weiteren haben die erfindungsgemäß verwendeten Wirkstoffe der allgemeinen Formel (I) den Vorteil, daß sie keine Kreuzresistenzen zu bereits etablierten Medikamenten bzw. Chemotherapeutika zeigen.

Als nicht beschränkende Beispiele für Tumor- bzw. Krebserkrankungen, für deren Behandlung die Verbindungen der allgemeinen Formel (I) eingesetzt werden können, lassen sich die folgenden Erkrankungen nennen: Darmkrebs (Kolonkarzinome), Brustkrebs (Mammakarzinome), Ovarialkarzinome, Uteruskarzinome, Lungenkrebs, Magenkrebs, Leberkrebs, Pankreaskarzinome, Nierenkrebs, Blasenkrebs, Prostatakrebs, Hodenkrebs, Knochenkrebs, Hautkrebs, Kaposi-Sarkome, Hirntumore, Myosarkome, Neuroblastome (z. B. Retinoblastome), Lymphome und Leukämien.

Erfindungsgemäß bevorzugt ist die Verwendung der folgenden Verbindungen der Formeln (Ia), (Ib), (Ic) und (Id) einschließlich ihrer physiologisch verträglichen Salze, Hydrate, Isomeren, insbesondere Stereoisomeren, Tautomeren und Konstitutionsisomeren, Derivate, Prodrugs und Metabolite:

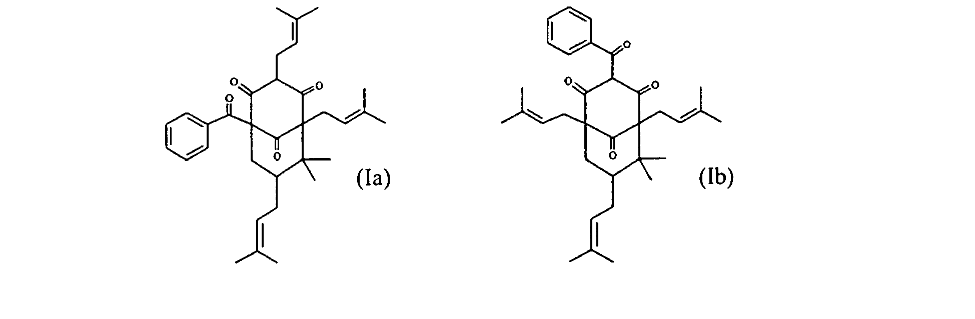

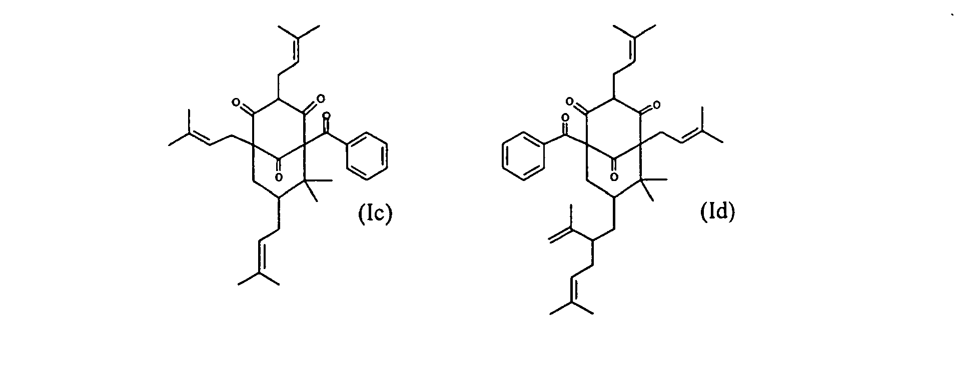

Erfindungsgemäß besonders bevorzugt ist die Verwendung der folgenden Verbindung der allgemeinen Formel (Ic) einschließlich ihrer physiologisch verträglichen Salze, Hydrate, Isomeren, insbesondere Stereoisomeren, Tautomeren und Konstitutionsisomeren, Derivate, Prodrugs und Metabolite:

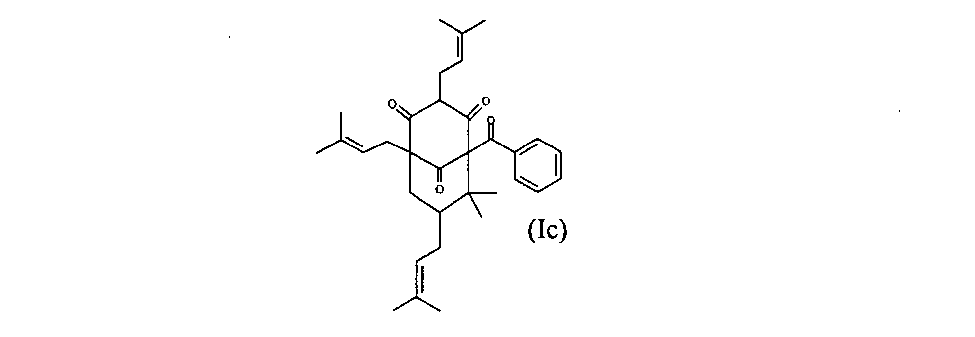

Die Verbindung der allgemeinen Formel (Ic) ist eines von mehreren Keto/Enol-Tautomeren, welche im Gleichgewicht nebeneinander vorliegen:

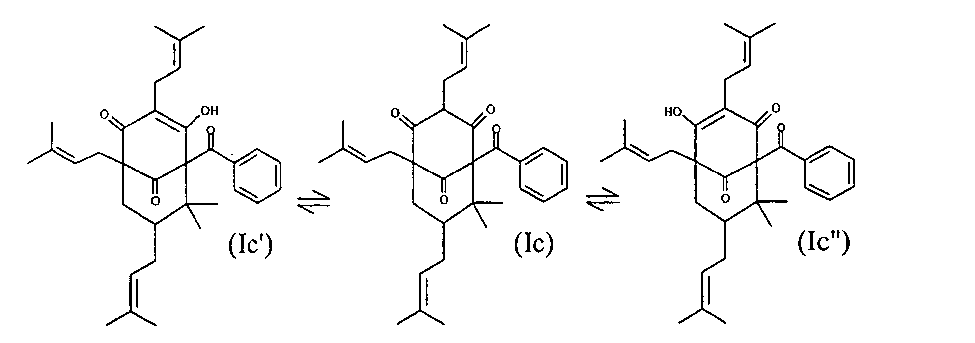

Erfindungsgemäß ganz besonders bevorzugt ist es, wenn die Verbindung der allgemeinen Formel (Ic) die folgende Konfiguration aufweist.

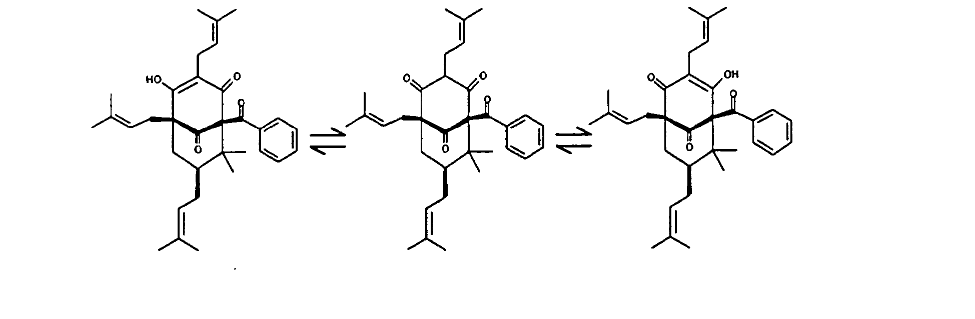

Der Anmelder hat weiterhin herausgefunden, daß es in bestimmten Fällen vorteilhaft sein kann, die Verbindungen der allgemeinen Formel (I) und/oder ihre physiologisch verträglichen Salze, Hydrate, Isomere, insbesondere Stereoisomere, Tautomere und Konstitutionsisomere, Derivate, Prodrugs und Metabolite in Kombination mit mindestens einem weiteren Chemotherapeutikum, insbesondere einem Proteinkinaseinhibitor, vorzugsweise einem MAP-Kinase-Inhibitor, einzusetzen bzw. zu verabreichen. Besonders gute Ergebnisse in der Therapie von Krebs- und Tumorerkrankungen können mit einer Kombination aus Verbindungen der allgemeinen Formel (I) und/oder ihrer physiologisch verträglichen Salze, Hydrate, Isomeren, insbesondere Stereoisomeren, Tautomeren und Konstitutionsisomeren, Derivaten, Prodrugs und Metabolite einerseits und einem Proteinkinaseinhibitor, vorzugsweise MAP-Kinase-Inhibitor, andererseits erzielt werden. Derartige Kombinationen wirken überraschenderweise synergistisch.

Gegenstand der vorliegenden Erfindung ist somit auch eine pharmazeutische Kombination, insbesondere cytostatische Kombination, die
(A) mindestens eine Verbindung der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite; und
(B) mindestens ein weiteres Chemotherapeutikum, insbesondere einen Proteinkinaseinhibitor, vorzugsweise MAP-Kinase-Inhibitor,
umfaßt.

Die einzelnen Komponenten (A) und (B) der erfindungsgemäßen Kombination können entweder gleichzeitig oder aber zeitlich abgestuft angewandt bzw. verabreicht werden. Die pharmazeutische Kombination kann die Komponenten (A) und (B) entweder als funktionelle Einheit, insbesondere in Form einer Mischung, eines Gemisches oder eines Gemenges, oder aber (räumlich) getrennt voneinander umfassen.

Proteinkinaseinhibitoren wirken, wie ihr Name schon sagt, inhibierend in bezug auf die Aktivität von Proteinkinasen, d. h. Enzyme, welche die γ-Phosphatgruppen von ATP auf Tyrosin- oder Serin- und Threonin-Seitenketten übertragen. Für die erfindungsgemäße pharmazeutische Kombination und die erfindungsgemäße Kombinationstherapie insbesondere von Tumor- bzw. Krebserkrankungen eignen sich Proteinkinaseinhibitoren aller Art, z. B. alle aus dem Stand der Technik bekannten Proteinkinaseinhibitoren (z. B. bestimmte niedermolekulare heterocyclische Inhibitoren wie Staurosporin, 2'-Amino-3'-methoxyflavon, 1,4-Diamino-2,3-dicyano-1,4-[2-aminophenylthio]-butadien, SB 203580 von SmithKline Beecham etc.). Hierbei kann ein synergistischer Effekt beobachtet werden.

Für die erfindungsgemäße pharmazeutische Kombination und die erfindungsgemäße Kombinationstherapie von Tumor- bzw. Krebserkrankungen besonders bevorzugt ist die Verwendung von MAP-Kinase-Inhibitoren. MAP-Kinase-Inhibitoren wirken, wie ihr Name schon sagt, inhibierend in bezug auf die Aktivität von MAP-Kinasen (MAP = mitogen activated protein), d. h. Enzyme, welche insbesondere bei der Mitose aktiv sind und die endständigen Phosphatreste von Nucleotidtriphosphaten auf entsprechende Substrate übertragen. Auf diese Weise verhindern MAP-Kinase-Inhibitoren die Mitose und damit die Zellteilung von Krebs- bzw. Tumorzellen. Für die erfindungsgemäße pharmazeutische Kombination und die erfindungsgemäße Kombinationstherapie insbesondere von Tumor- bzw. Krebserkrankungen eignen sich MAP-Kinase-Inhibitoren aller Art, z. B. alle aus dem Stand der Technik bekannten MAP-Kinase-Inhibitoren. Beispiele für erfindungsgemäß einsetzbare MAP-Kinase-Inhibitoren sind z. B. in den folgenden Druckschriften genannt WO 99/32111 A, WO 99/64400 A, WO 98/52941 A, WO 98/52937 A, WO 97/44467 A, WO 98/27098 A, WO 98/52558 A, WO 98/52940 A, WO 99/32110 A, WO 99/32463 A, WO 99/58502 A, WO 99/58523 A und WO 99/00357 A.

Des weiteren hat der Anmelder überraschend gefunden, daß die zuvor genannten Verbindungen der allgemeinen Formel (I) auch eine regulatorische Aktivität innerhalb der MAP-Kinase-Signaltransduktion zeigen, d. h. als Regulatoren innerhalb der MAP-Kinase-Signaltransduktion wirken.

Weiterhin betrifft die vorliegende Erfindung auch die Verwendung der Verbindungen der allgemeinen Formel (I) einschließlich ihrer physiologisch verträglichen Salze, Hydrate, Isomeren, insbesondere Stereoisomeren, Tautomeren und Konstitutionsisomeren, Derivate, Prodrugs und Metabolite in therapeutisch wirksamen Dosen bzw. Gaben, gegebenenfalls auch in Kombination mit weiteren Wirkstoffen, insbesondere Chemotherapeutika (z. B. Proteinkinaseinhibitoren wie beispielsweise MAP-Kinase-Inhibitoren) zur Herstellung eines Arzneimittels zur Prophylaxe und/oder Behandlung von Erkrankungen des menschlichen oder tierischen Körpers, insbesondere Tumor- bzw. Krebserkrankungen.

Die erfindungsgemäß eingesetzten Wirkstoffe bzw. Wirkstoffkombinationen können, je nach Art der zur behandelnden Erkrankungen, systemisch oder aber auch topisch, insbesondere lokal, appliziert werden.

Für die Applikation der erfindungsgemäß eingesetzten Wirkstoffe bzw. Wirkstoffkombinationen kommen alle üblichen Applikationsformen in Betracht. Beispielsweise kann die Applikation oral, lingual, sublingual, bukkal, rektal oder parenteral (d. h. unter Umgehung des Intestinaltraktes, also intravenös, intraarteriell, intrakardial, intrakutan, subkutan, transdermal, intraperitoneal oder intramuskulär) erfolgen, insbesondere geeignet sind die orale und die intravenöse Applikation; ganz besonders bevorzugt ist die orale Applikation. Weiterhin ist eine topische Anwendung möglich (z. B. zur Behandlung von Melanomen).

Für die erfindungsgemäße Anwendung werden die Wirkstoffe bzw. Wirkstoffkombinationen in bekannter Weise in die üblichen Formulierungen überführt, wie z. B. Tabletten, Dragees, Pillen, Granulate, Aerosole, Sirupe, Emulsionen, Suspensionen, Lösungen, Salben, Cremes und Gele aller Art, insbesondere unter Verwendung inerter, im wesentlichen nichttoxischer, pharmazeutisch geeigneter Trägerstoffe oder Lösungsmittel. Des weiteren ist es möglich, die eingesetzten Wirkstoffe bzw. Wirkstoffkombinationen quasi in liposomale "Verpackungen" einzubringen, d. h. quasi eingebettet oder verkapselt in Liposomen, die ihrerseits gegebenenfalls weiter modifiziert sein können (z. B. verestert mit PEG). Hierbei können die erfindungsgemäß eingesetzten Wirkstoffe bzw. Wirkstoffkombinationen jeweils in einer therapeutisch wirksamen Konzentration, insbesondere in Konzentrationen von etwa 0,0001 bis etwa 99 Gew.-%, bevorzugt etwa 0,01 bis etwa 95 Gew.-%, der Gesamtmischung vorhanden sein, d. h. in Mengen, die ausreichend sind, um den angegebenen bzw. gewünschten Dosierungsspielraum zu erreichen. Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über einen definierten Zeitraum, z. B. über den Tag, zu verteilen.

Die Formulierungen werden beispielsweise hergestellt durch Verstrecken der Wirkstoffe bzw. Wirkstoffkombinationen mit Lösungsmitteln (z. B. Öle wie Rizinusöl) und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Je nach Applikationsart hat es sich als vorteilhaft erwiesen, die erfindungsgemäß eingesetzten Wirkstoffe bzw. Wirkstoffkombinationen in Mengen von etwa 0,0001 bis etwa 500 mg/kg Körpergewicht, insbesondere etwa 0,0001 bis etwa 100 mg/kg, vorzugsweise 0,01 bis 50 mg/kg, zur Erzielung wirksamer Ergebnisse zu verabreichen. Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese über einen definierten Zeitraum, z. B. über den Tag, zu verteilen, und zwar z. B. in mehreren Einzelgaben oder als Dauerapplikation (z. B. Dauerinfusion). Gleichermaßen ist die Anwendung in einer chronischen Therapie (z. B. in Tablettenform) möglich.

Die vorliegende Erfindung wird anhand der folgenden Ausführungsbeispiele veranschaulicht, welche die vorliegende Erfindung jedoch keinesfalls beschränken.

### AUSFÜHRUNGSBEISPIELE

### 1-Benzoyl-8,8-dimethyl-2-hydroxy-3,5,7-tris-(3-methyl-2-butenyl)-bicyclo-[3.3.1]non-2-en-4,9-dion (Ic') aus dem Propolisextrakt karibischer Bienen

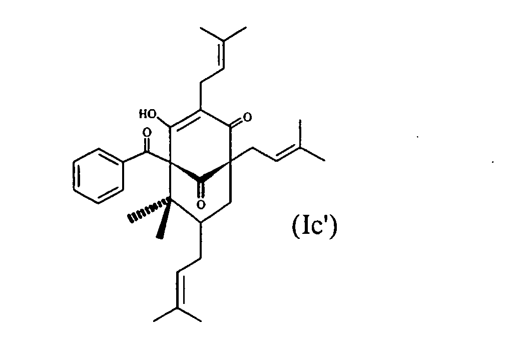

Im folgenden wird die Isolierung der Verbindung der Formel (Ic') und ihre physiologische Wirksamkeit beschrieben.

Die Verbindung der Formel (Ic') liegt im tautomeren Gleichgewicht mit den Strukturen der Formeln (Ic) und (Ic") vor:

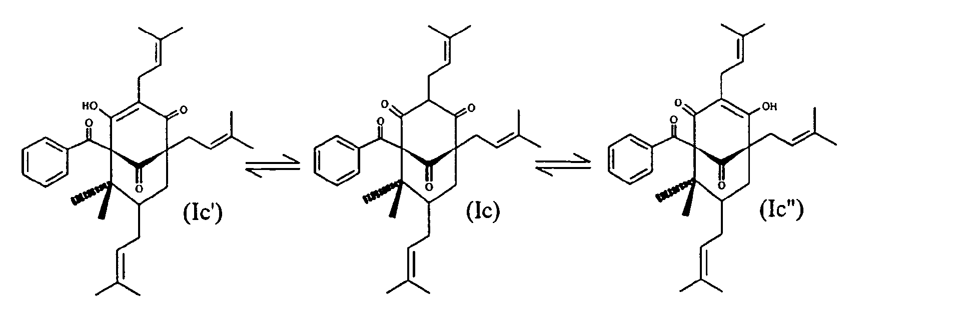

Der Einfachheit halber wird im folgenden aber nur noch von der Verbindung der Formel (Ic') die Rede sein.

### Herstellung von Propolis-Lösungen

Propolis, gesammelt von Bienenstöcken in der Karibik, wird in Ethanol aufgenommen, die unlöslichen Wachse mittels Filtration abgetrennt. Definierte Volumina werden getrocknet, das resultierende Pulver zu einer definierten Konzentration in Ethanol resuspendiert. Die Lösungen werden bei Raumtemperatur unter Lichtausschluß gelagert.

### Semipräparative HPLC-Fraktionierung der Propolis-Lösungen

Die Fraktionierung erfolgt mit einer RP-HPLC-Anlage der Firma Waters GmbH (Eschborn) (Waters the Separator Module Alliance 2690, Waters PDA 996 Detektor, Waters Millennium Chromatography Manager). Die Fraktionierung gelingt auf einer Trennsäule der Firma Macherey-Nagel (Nucleosil 100-7 C18, 250 x 21 mm) unter Benutzung eines Gradientensystems bei 40 °C (Tabelle 1). Die wäßrige Phase besteht aus einer 0,01 M Ammoniumformiatlösung (pH = 7.00).

**Tabelle 1: Gradientensystem**

| **Zeit (min)** | **Flußrate (ml/min)** | **Ammoniumformiat %** | **Methanol %** | **Acetonitril %** |
|---|---|---|---|---|
| 0 | 4 | 50 | 30 | 20 |
| 80 | 4 | 10 | 70 | 20 |
| 180 | 4 | 0 | 80 | 20 |
| 190 | 4 | 0 | 80 | 20 |
| 200 | 4 | 50 | 30 | 20 |
| 210 | 4 | 50 | 30 | 20 |

Die so gewonnenen Fraktionen zu je 8 ml (2 min) werden getrocknet und in Methanol resuspendiert und unter Ausschluß von Licht bei Raumtemperatur gelagert.

Zur Überprüfung der Cytotoxizität der einzelnen Fraktionen werden vergleichbare Volumina in einem Medium verdünnt und in dem im folgenden beschriebenen SRB-Assay untersucht. Als Zielzelle wird unter anderem eine Darmkrebszell-Linie humanen Ursprungs (HCT8 WT) verwendet.

### Reinigung der cytotoxischen Fraktion

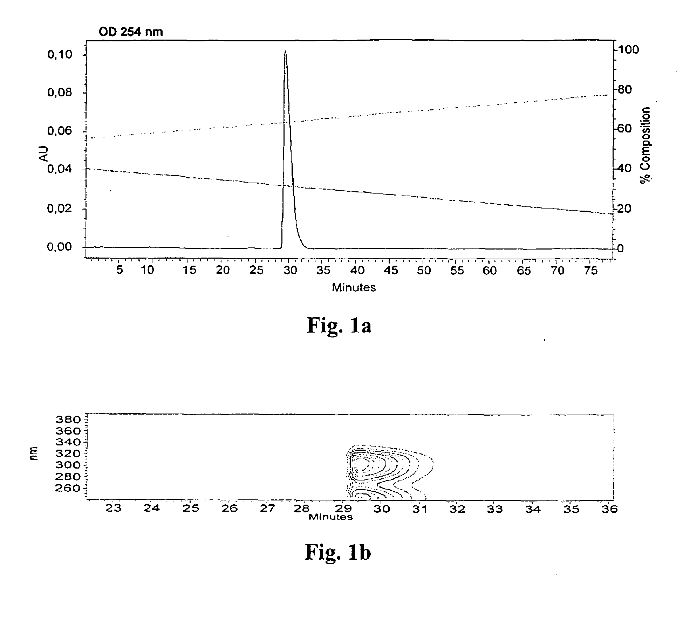

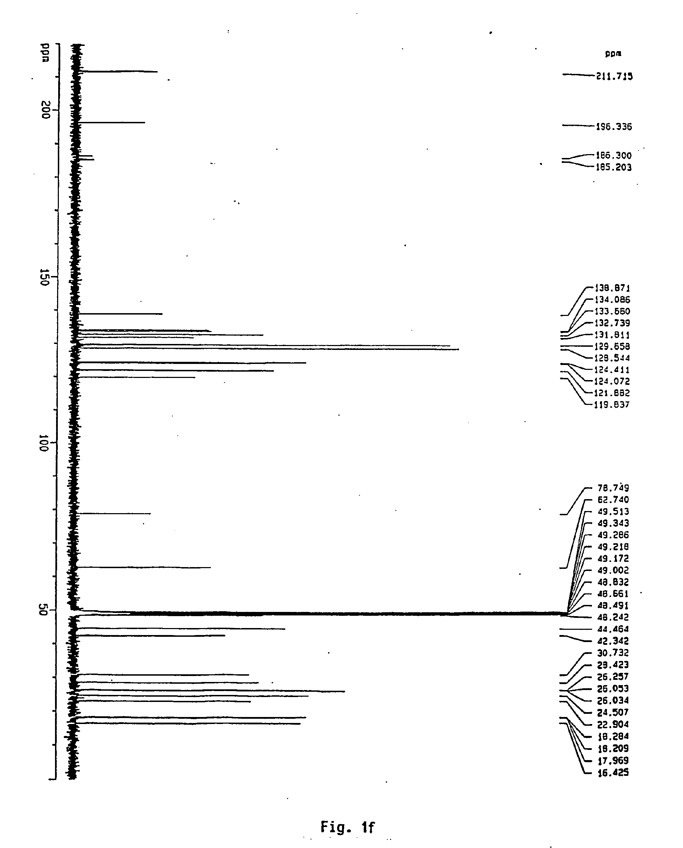

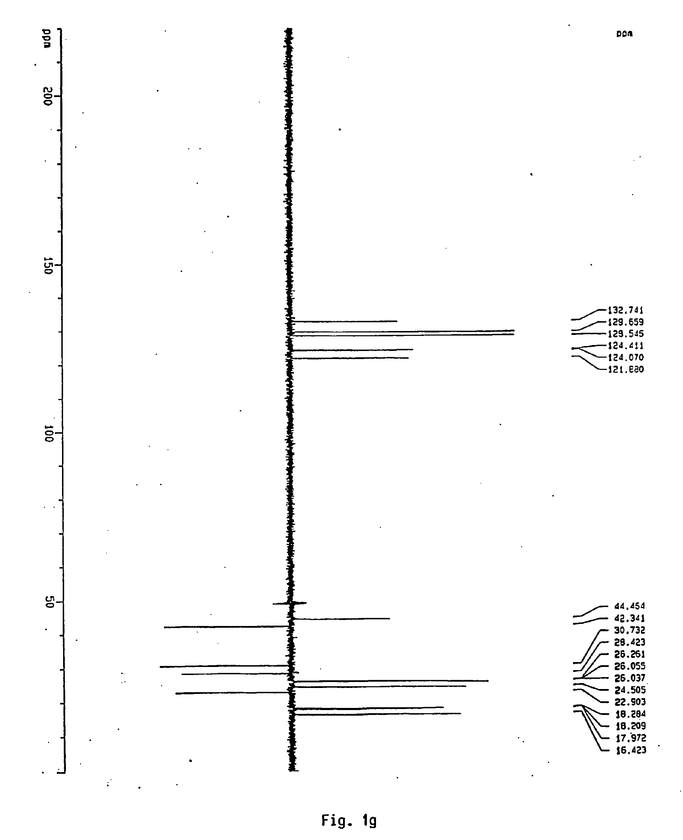

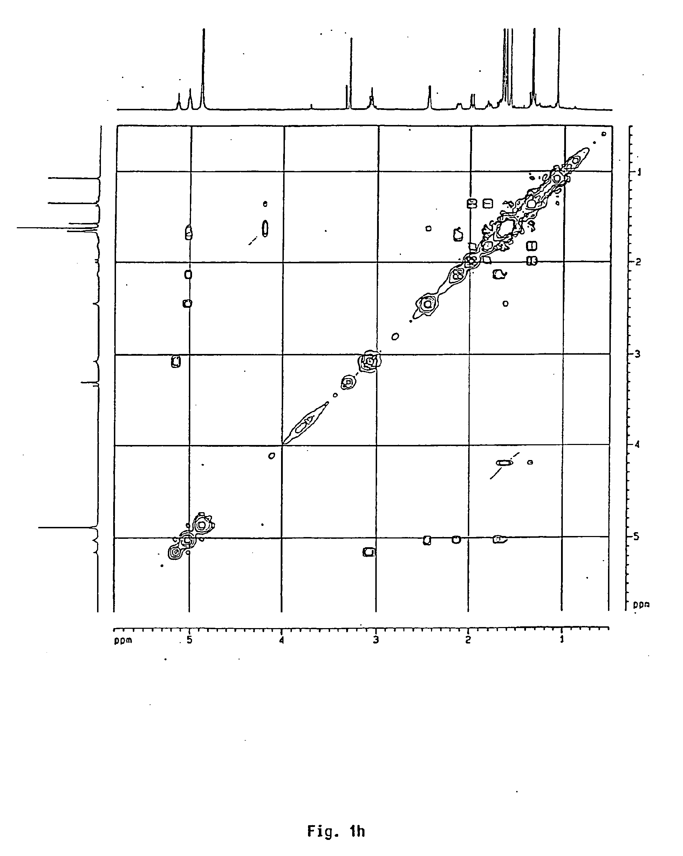

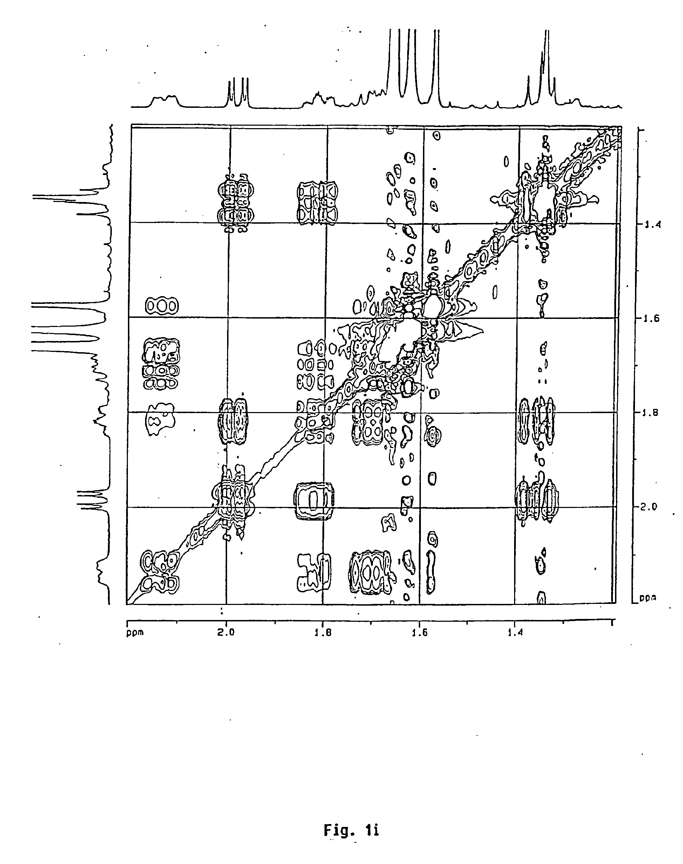

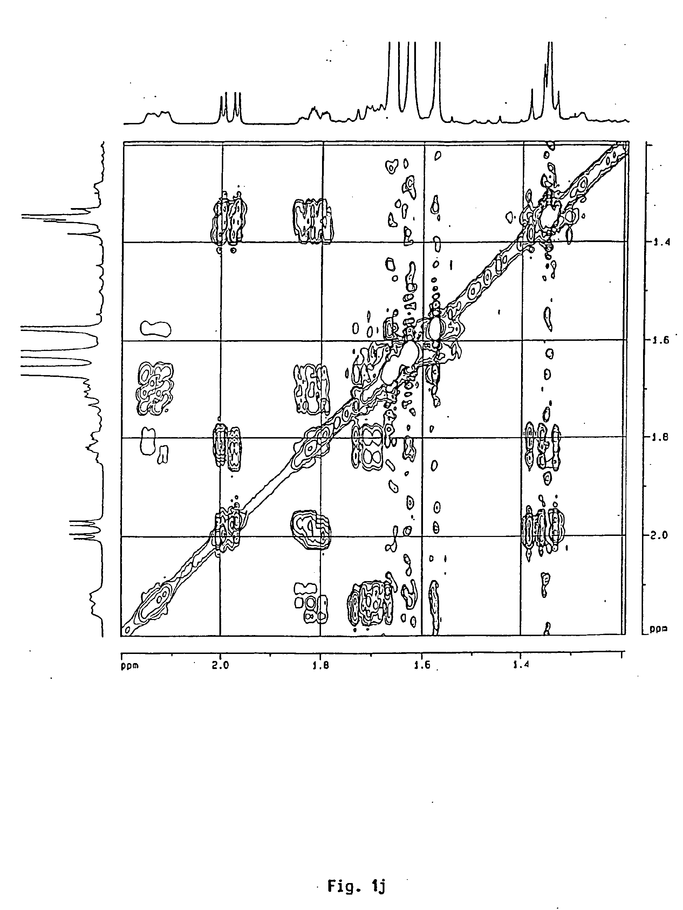

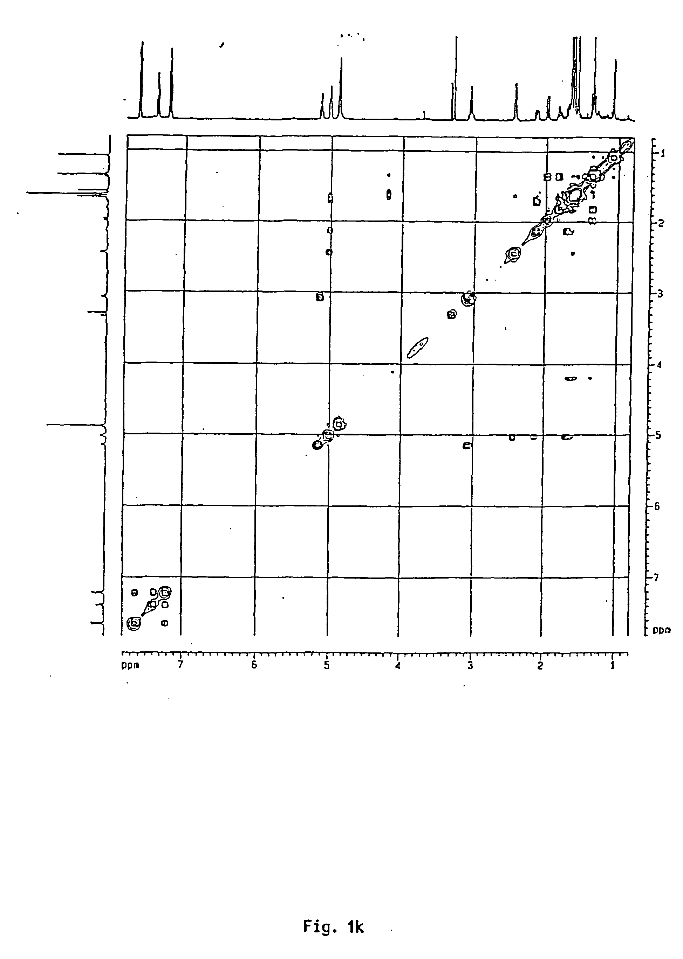

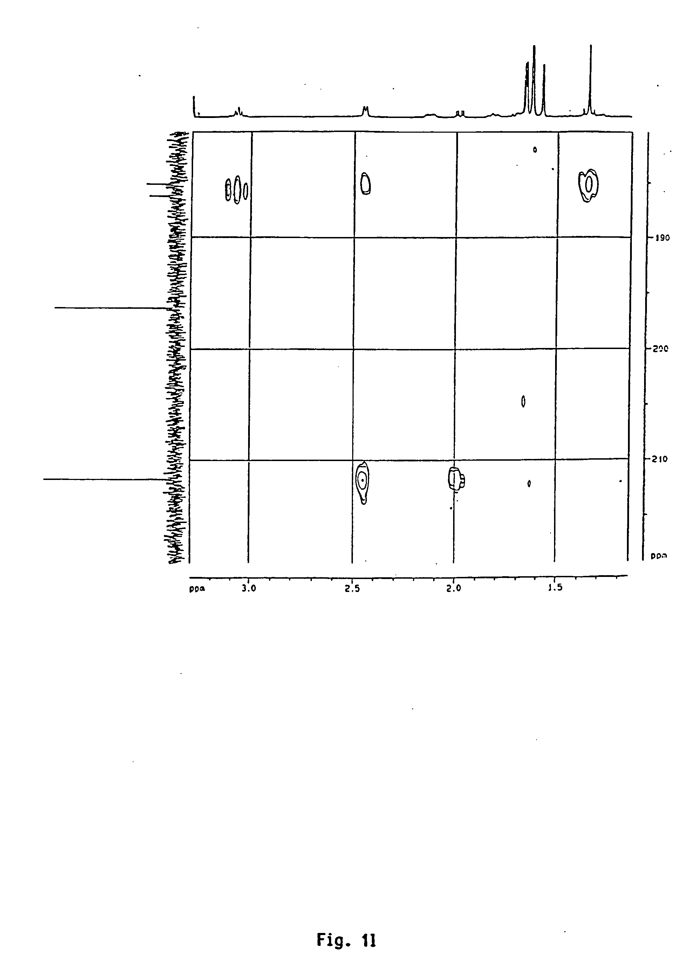

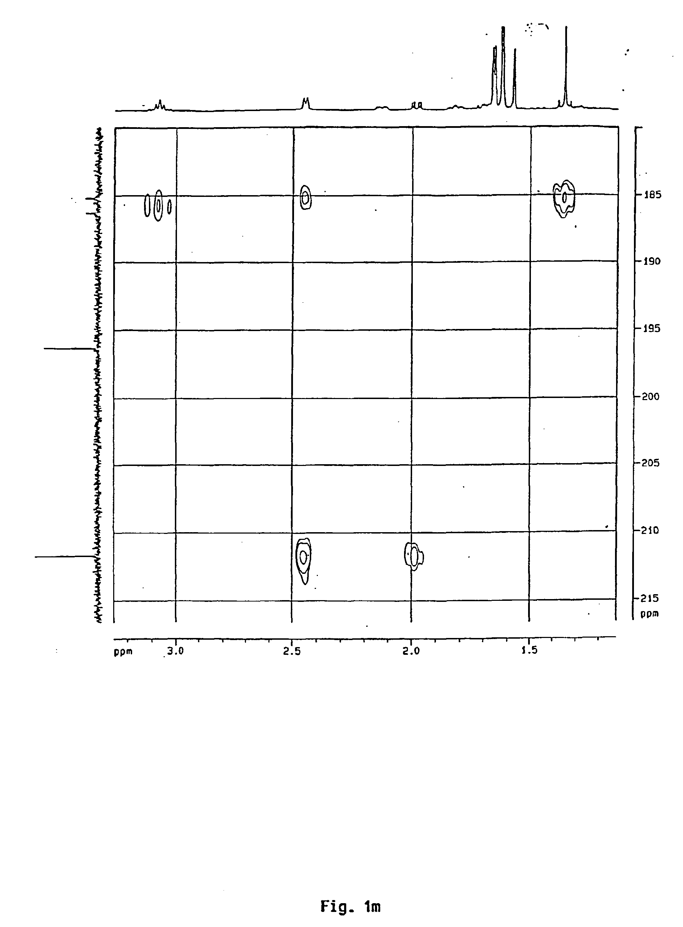

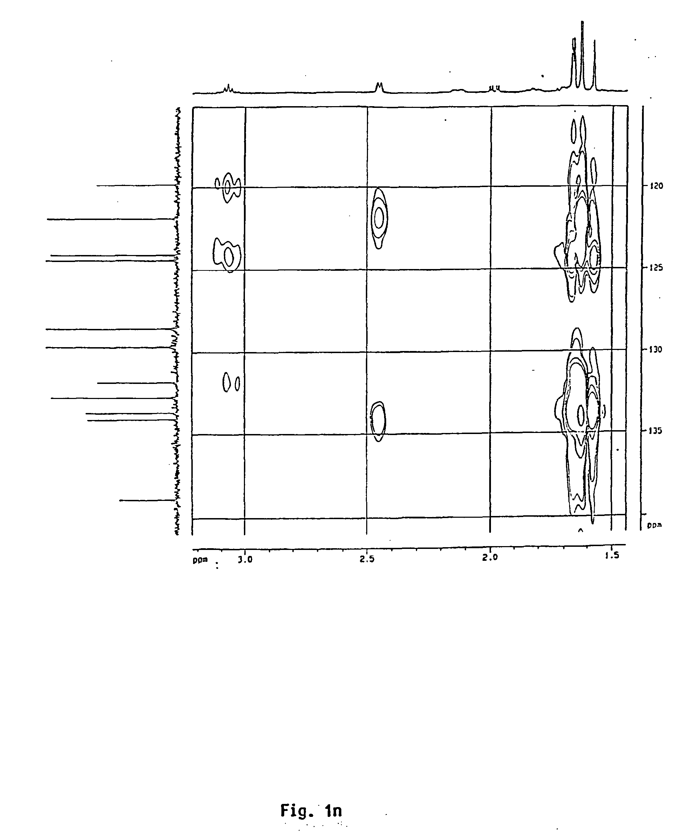

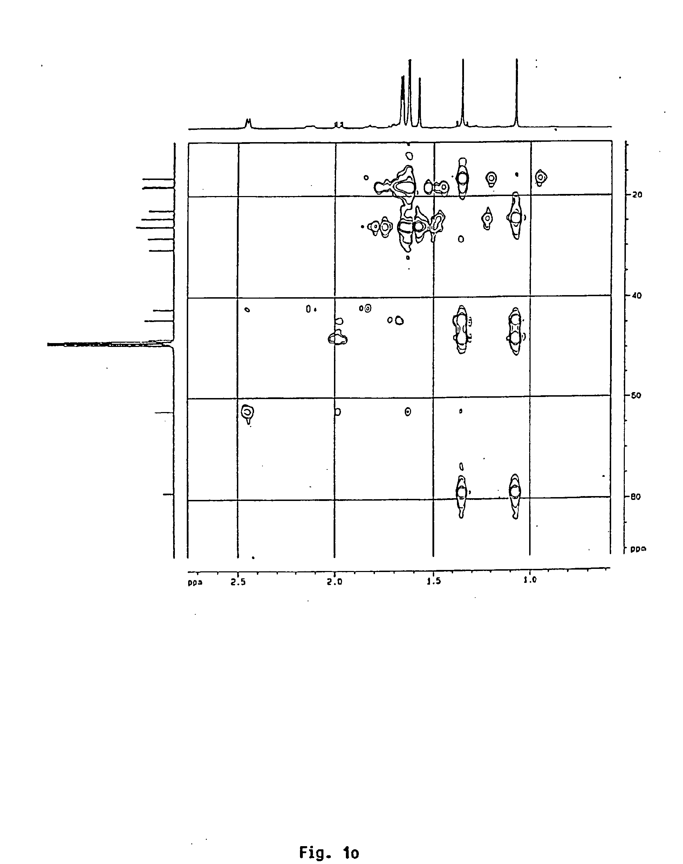

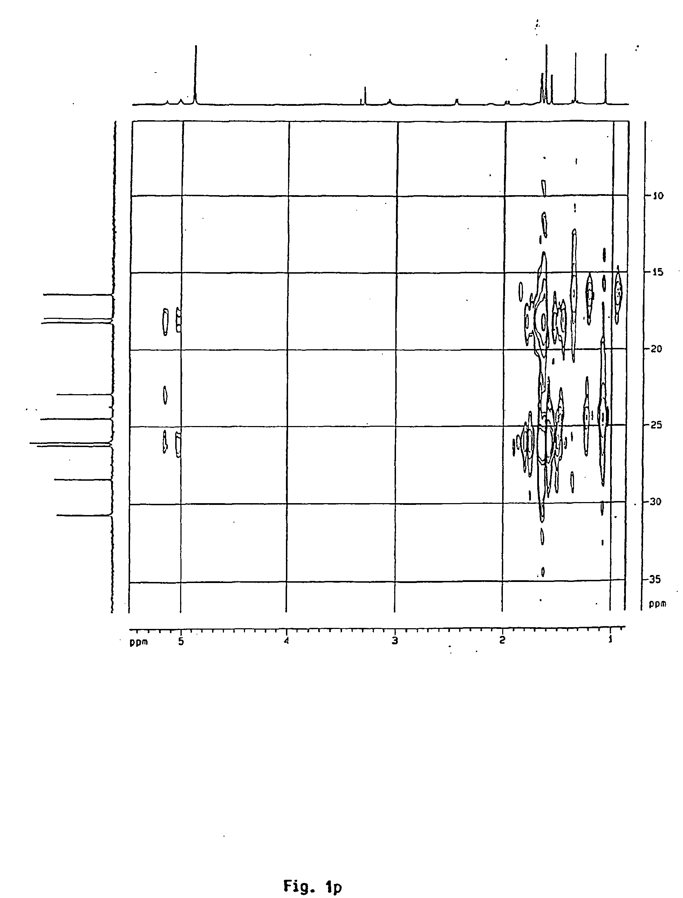

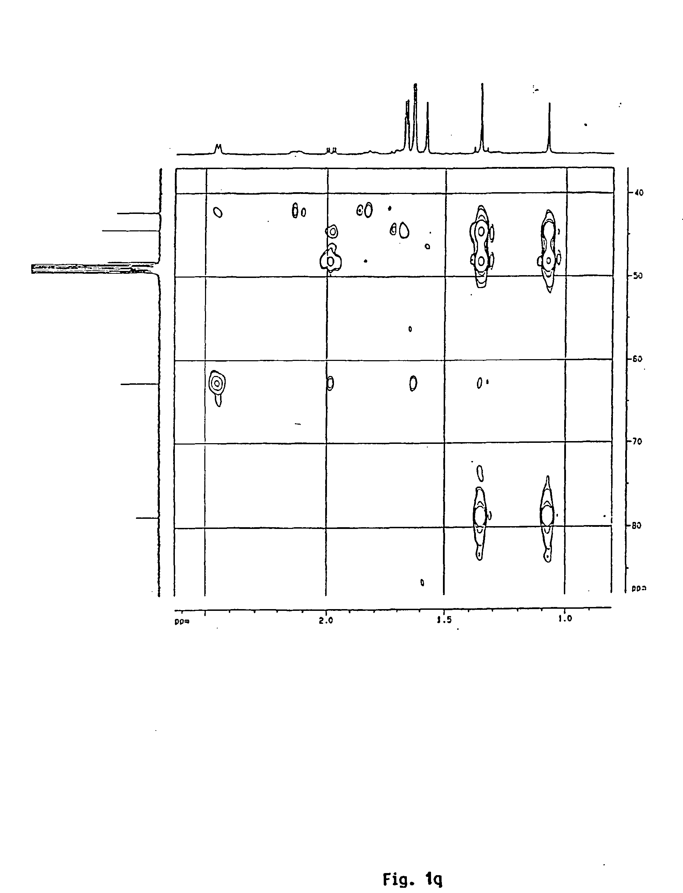

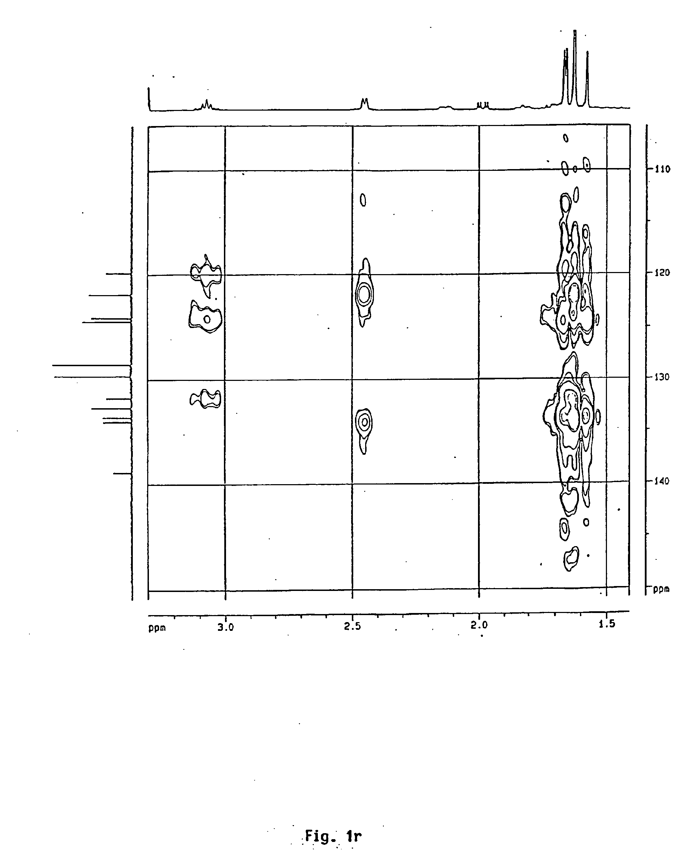

Die im SRB-Test cytotoxisch wirkende Fraktion wird einer weiteren Reinigung unterzogen. Dazu wird das Gradientenelutionssystem der Polarität der zu reinigenden Fraktion angepaßt. Nach erneuter Fraktionierung und Überprüfung der mittels präparativer RP-HPLC erhaltenen Fraktionen im SRB-Test kann eine einheitliche Substanz (Ic') isoliert werden. Die Reinheit der Substanz (Ic') wird anhand UV-spektroskopischer Daten belegt (Fig. 1b). Als analytische Trennsäule wird eine Waters Symmetry Säule, gefüllt mit einer modifizierten C18-Phase (250 mm x 4,6 mm), gewählt. Das Ergebnis ist in Fig. 1a dargestellt. '
Fig. 1a zeigt ein repräsentatives Chromatogramm der Substanz (Ic') aus der analytischen RP-HPLC (Reinheitsüberprüfung) bei 254 nm, Flußrate = 1 ml/min, T = 40 °C, Gradientensystem wie angegeben. Die obere Linie entspricht der Konzentration an Methanol (%), die mittlere Linie der Konzentration an wäßriger Phase (%, Ammoniumformiat 0,01M). Die Konzentration an Acetonitril (%) wird konstant auf 5 % gehalten (untere Linie).
Fig. 1b zeigt die PDA-Daten der in Fig. 1a gezeigten Analytik. Die Substanz (Ic') wird als einheitliche Fraktion gewonnen. Das UV-Spektrum unter diesen Elutionsbedingungen weist zwei Maxima auf (254 und 308 nm).

### Strukturaufklärung

Die erste Isolierung und Charakterisierung der Verbindung der allgemeinen Formel (Ic') erfolgt ohne weitere Derivatisierung (z. B. mit Diazomethan). Die ethanolischen Extrakte von Propolis (gesammelt in der Karibik) können, wie zuvor beschrieben, mittels RP-HPLC Analytik gereinigt werden, wodurch die Verbindung der Formel (Ic') erstmals in seiner nichtderivatisierten Form zur Verfügung für weitere NMR-spektroskopische Untersuchungen gestellt werden kann, und zwar in ausreichend hoher Reinheit (> 98 %; LC-MS; UVspektroskopische Untersuchungen mittels LC-PDA; MS).
Zur weiteren Bestätigung der Struktur wird die Verbindung der Formel (Ic') durch Umsetzung mit Diazomethan in die korrespondierenden Methylether überführt. Die O-methylierten Verbindungen werden im Verhältnis 40/60 isoliert (RP-HPLC).

Die Ergebnisse der NMR-Untersuchungen (¹H-NMR, ¹³C-NMR, DEPT135, DEPT90, HMQC, HMBC, H,H-COSY90 (gs), 1D-NOE-Differenzspektrum) sind im folgenden tabellarisch angegeben:
- DEPT steht für **D**istortionless **E**nhancement by **P**olarization **T**ransfer.
- COSY steht für **CO**rrelated **S**pectroscop**Y**. Dabei ist im allgemeinen das ¹H,¹H-COSY gemeint, welches nach seinem "Erfinder" auch Jeener-Experiment genannt wird und die erste 2D-Impulssequenz in der Geschichte der NMR darstellt.
- HMQC steht für **H**eteronuclear **M**ultiple **Q**uantum **C**oherence.
- HMBC steht für **H**eteronuclear **M**ultiple-**B**ond **C**orrelation.
- NOE steht für **N**uclear **O**verhauser **E**ffekt.

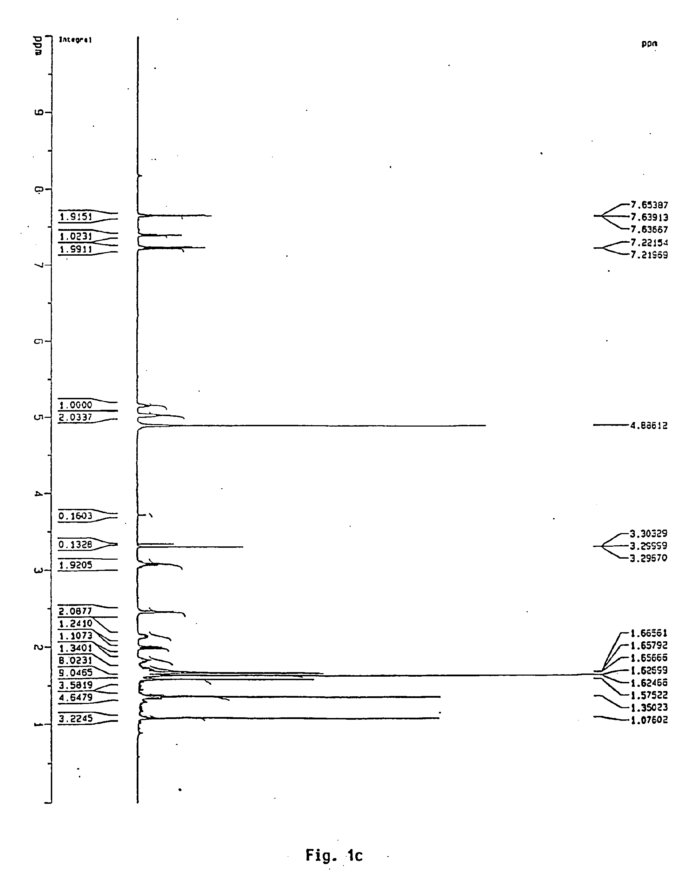

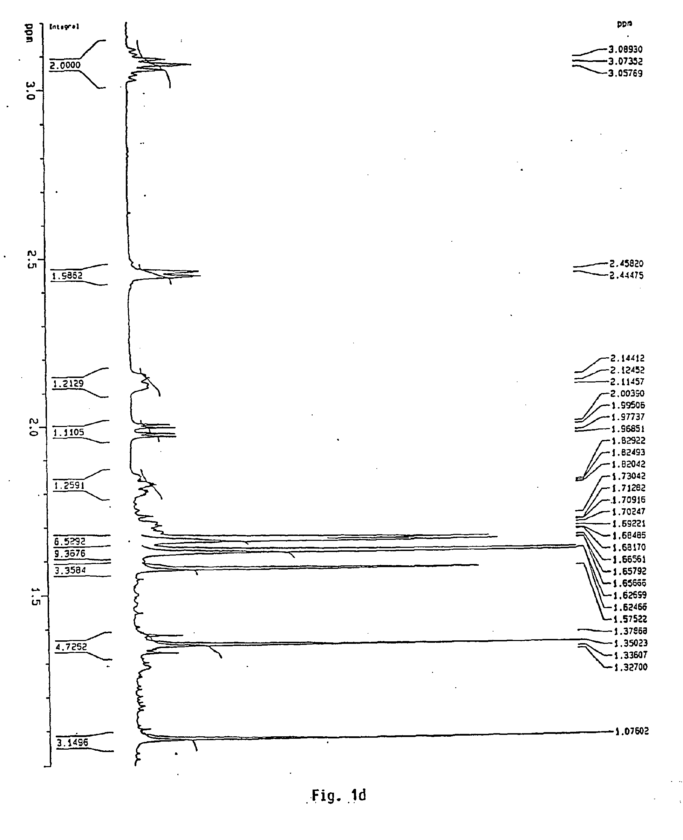

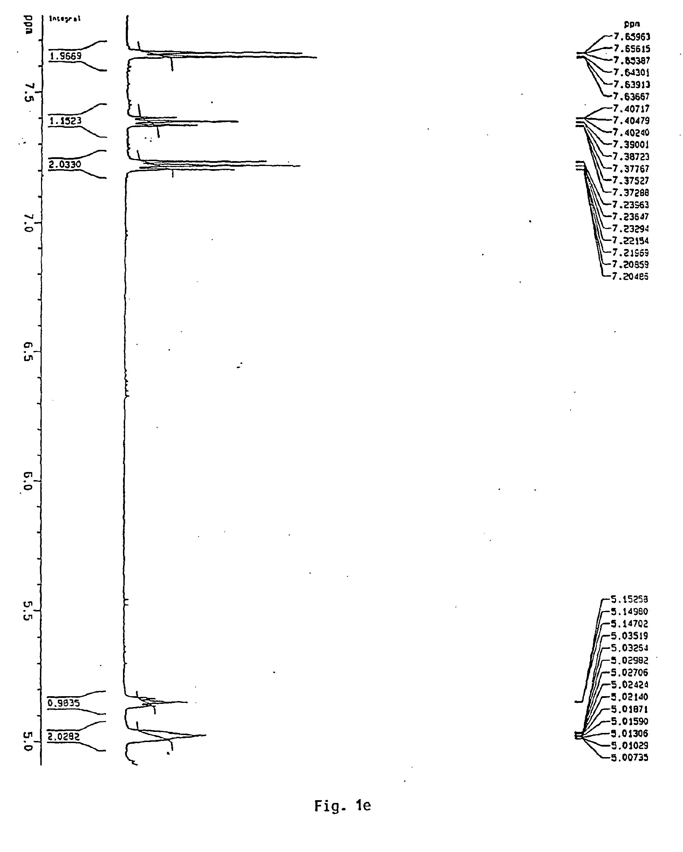

Fig. 1c, 1d und 1e sind ¹H-NMR-Spektren der Verbindung (Ic') (Meßfrequenz: 500 MHz, Lösemittel: CD₃OD).

Fig. 1 f stellt ein ¹³C-NMR-Spektrum der Verbindung (Ic') (Meßfrequenz: 125 MHz, Lösemittel: CD₃OD) dar.

Fig. 1g stellt ein ¹³C-DEPT-Spektrum der Verbindung (Ic') (Meßfrequenz: 125 MHz, Lösemittel: CD₃OD) dar; die CHs und CH₃s zeigen nach oben, während die CH₂s nach unten zeigen.

Fig. 1h, 1i, 1j und 1k sind H,H-COSY-Spektren der Verbindung (Ic') (Meßfrequenz: 500 MHz, Lösemittel: CD₃OD).

Fig. 11 bis 1r sind HMBC-Spektren der Verbindung (Ic') (Meßfrequenz: 125 MHz, Lösemittel: CD₃OD).

**Tabelle 1a: NMR-Daten der Verbindung (Ic')**

| Verbindung der Formel (Ic') in CD₃OD | | | |
|---|---|---|---|
| C# | δ ¹³C, ppm | HMBC Korrel. mit H# | δ ¹H, ppm |
| 1 | 78.75 | 27,28 | - |
| 2 | 186.30 (br) | 17 | - |
| 3 | 119.83 | 17 | - |
| 4 | 185.20 (br) | 22, 17, 6a | - |
| 5 | 62.74 | 22, 6a, 6e | - |
| 6 | 42.34 | 7,22,29 | 1.98 (1H, dd, 4.5 u. 13.1 Hz, eq) |
| | | | 1.35 (1H, t, overlap, 13.1 Hz, ax.) |
| 7 | 44.46 | 27,28 | 1.83 |
| 8 | 48.24 | 6e, 27, 28 | - |
| 9 | 211.72 | 22, 6e | - |
| 10 | 196.39 | 12, 16 | - |
| 11 | 138.87 | 13, 15 | - |
| 12 | 129.66 | 14, 16 | 7.65 (1H) |
| 13 | 128.54 | 15 | 7.22 (1 H) |
| 14 | 132.74 | 12, 16 | 7.39 (1H) |
| 15 | 128.54 | 13 | 7.22 (1H9 |
| 16 | 129.66 | 12, 14 | 7.65 (1H) |
| 17 | 22.9 | 18(w) | 3.10 (1H, dd, J=14 u. 7 Hz) |
| | | | 3.05 (1H, dd, J=14, u. 7 Hz) |
| 18 | 124.07 | 17(w), 20, 21 | 5.15 (1H, tm, J=7 u. 1.4 Hz) |
| 19 | 131.81 | 20 | - |
| 20 | 26.26 | 18,21 | 1.68 (3H, s) |
| 21 | 17.96 | 18,20 | 1.63 (3H, s) |
| 22 | 30.73 | | 2.47 (1H, dd, J=15 u. 7 Hz) |
| | | | 2.44 (1H, dd, J=15 u. 7 Hz) |
| 23 | 121.88 | 22,25,26 | 5.02 (1 H, m) |
| 24 | 134.09 | 22, 25, 26 | - |
| 25 | 26.03 | 23, 26 | 1.63 (3H, s) |
| 26 | 18.28 | 23,25 | 1.63 (3H, s) |
| 27 | 24.51 | 28 | 1.35 |
| 28 | 16.42 | 27 | 1.08 |
| 29 | 28.42 | | 2.13 (1H, m) |
| | | | 1.70 (1H, m, overlap) |
| 30 | 124.41 | 32,33 | 5.02 (1H, m) |
| 31 | 133.68 | 32, 33 | - |
| 32 | 26.05 | 30,33 | 1.58 (3H, s) |
| 33 | 18.20 | 30, 32 | 1.67 (3H, s) |

| | | | |
|---|---|---|---|
| H,H-COSY: H6a-H7, H6e-H7, H7-H29, H7-H29' (w) = weak, (br) = broad | | | |

Dementsprechend liegt die nichtderivatisierte Verbindung der Formel (Ic') bzw. (Ic") bzw. (Ic) in einem Keto/Enol-Gleichgewicht vor, was sowohl durch die chemische Verschiebung der Signale, als auch durch die Breite der ¹³C-Signale der C-Atome 2 und 4 bestätigt wird.

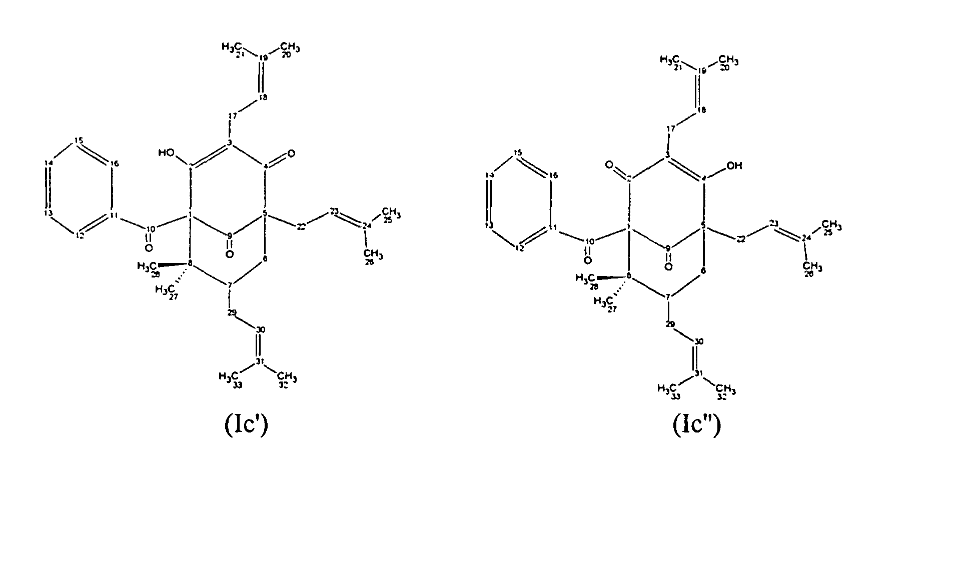

**Tabelle 1b: NMR-Daten des Methylethers (II)**

| Verbindung der Formel (II) in CDCl₃ | | | |
|---|---|---|---|
| C# | δ ¹³C, ppm | HMBC Korrel. mit H# | δ ¹H, ppm |
| 1 | 79.25 | 27,28 | - |
| 2 | 194.07 | 17 | - |
| 3 | 126.80 | 17 | - |
| 4 | 173.07 | 17, 22, 6a, OMe | - |
| 5 | 59.81 | 22, 6a(w) | - |
| 6 | 40.22 | 22, 29(w) | 2.00 (1 H, dd, J=3.7 u. 13.4 Hz, eq) |
| | | | 1.45 (1H, dd, J=12.2 u. 13.4 Hz, ax) |
| 7 | 43.70 | 27, 28, 29, 6a(w) | 1.68 |
| 8 | 48.83 | 27, 28, 6e | - |
| 9 | 207.28 | 22, 6e | - |
| 10 | 193.06 | 12, 16 | - |
| 11 | 136.26 | 13, 15 | - |
| 12 | 128.57 | 14, 16 | 7.42 (1H) |
| 13 | 127.73 | 15 | 7.20 (1 H) |
| 14 | 131.89 | 12, 16 | 7.35 (1H) |
| 15 | 127.73 | 13 | 7.20 (1H) |
| 16 | 128.57 | 12,14 | 7.42 (1H) |
| 17 | 23.29 | | 3.23 (1 H, dd, J=15.2 u. 6.9 Hz) |
| | | | 3.14 (1H, dd, J=15.2 u. 6.9 Hz) |
| 18 | 121.32 | 17,20,21 | 5.00 (1H, m) |
| 19 | 133.03 | 17,20,21 | |
| 20 | 26.02 | 18, 21 | 1.67 (3H, s) |
| 21 | 18.09 | 18, 20 | 1.63 (3H, s) |
| 22 | 29.78 | | 2.57 (1H, dd, J=14.3 u. 7 Hz) |
| | | | 2.42 (1H, dd, J=14.3 u. 7 Hz) |
| 23 | 119.51 | 22,25,26 | 5.00 (1H, m) |
| 24 | 134.36 | 22, 25, 26 | - |
| 25 | 25.68 | 23,26 | 1.63 (3H, s) |
| 26 | 17.87 | 23,25 | 1.61 (3H, s) |
| 27 | 23.42 | 28 | 1.33 (3H, s) |
| 28 | 15.72 | 27 | 1.11 (3H,s) |
| 29 | 26.72 | | 2.14 (broad) (1H, dd, 6.5 u. 12.4 Hz) |
| | | | 1.70 (1H, overlap) |
| 30 | 122.58 | 32,33 | 4.98 (1H, m) |
| 31 | 133.49 | 32,33 | - |
| 32 | 25.79 | 30,33 | 1.66 (3H, s) |
| 33 | 17.87 | 30,32 | 1.56 (3H, s) |
| CH₃-O | 62.39 | | 3.99 (3H, s) |

| | | | |
|---|---|---|---|
| H,H-COSY: H6a-H7, H7-H29 (w) = weak | | | |

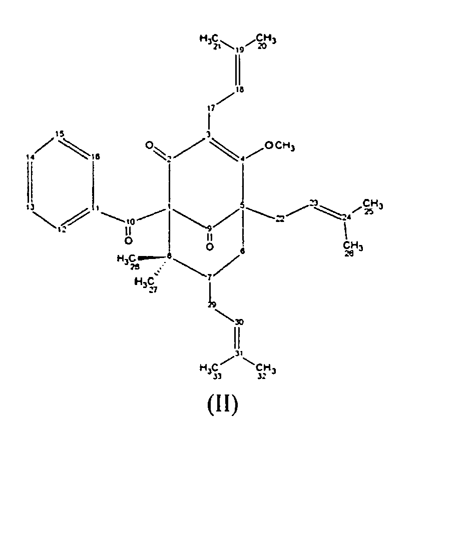

**Tabelle 1c: NMR-Daten des Methylethers (III)**

| Verbindung der Formel (III) in CDCl₃ | | | |
|---|---|---|---|
| C# | δ ¹³C, ppm | HMBC Korrel. mit H# | δ ¹H, ppm |
| 1 | 73.97 | 27,28 | - |
| 2 | 169.95 | 17,OMe | - |
| 3 | 123.22 | 17 | - |
| 4 | 197.14 | 17, 22, 6a | - |
| 5 | 65.17 | 7, 22 | - |
| 6 | 43.16 | 22, (w) | 1.92 (1H, dd, J=3.8 u. 12.9 Hz, eq) |
| | | | 1.42 (1H, t, J=12.9 Hz, ax) |
| 7 | 42.53 | 27, 28, 29, 6a | 1.66 |
| 8 | 47.86 | 27, 28, 6e | - |
| 9 | 207.91 | 22, 6e | - |
| 10 | 193.14 | 12, 16 | - |
| 11 | 137.03 | 13, 15 | - |
| 12 | 128.41 | 14, 16 | 7.59 (1H) |
| 13 | 127.94 | 15 | 7.28 (1H) |
| 14 | 132.08 | 12, 16 | 7.41 (1H) |
| 15 | 127.94 | 13 | 7.28 (1H) |
| 16 | 128.41 | 12,14 | 7.59 (1H) |
| 17 | 23.25 | 18 (w) | 3.32 (1H, dd, J=15.7 u. 6.8 Hz) |
| | | | 3.21 (1H, dd, J=15.7 u. 6.8 Hz) |
| 18 | 121.54 | 17,20,21 | 4.97 (1H, m) |
| 19 | 133.02 | 17,20,21 | |
| 20 | 25.85 | 18,21 | 1.66 (3H, s) |
| 21 | 18.02 | 18, 20 | 1.65 (3H, s) |
| 22 | 29.48 | | 2.55 (1H, dd, J=14 u. 7 Hz) |
| | | | 2.45 (1H, dd, J=14 u. 7 Hz) |
| 23 | 119.69 | 22,25,26 | 4.99 (1H, m) |
| 24 | 134.44 | 22, 25, 26 | - |
| 25 | 25.64 | 23, 26 | 1.66 (3H, s) |
| 26 | 18.15 | 23, 25 | 1.65 (3H, s) |
| 27 | 24.46 | 28 | 1.32 (3H, s) |
| 28 | 16.17 | 27 | 1.16 (3H,s) |
| 29 | 27.68 | | 2.07 (1H, m) |
| | | | 1.66 (1H, overlap) |
| 30 | 122.54 | 32,33 | 4.89 (1H, m) |
| 31 | 133.23 | 32,33 | - |
| 32 | 26.00 | 30, 33 | 1.65 (3H, s) |
| 33 | 17.86 | 30,32 | 1.53 (3H, s) |
| CH₃-O | 61.54 | | 3.44 (3H, s) |

| | | | |
|---|---|---|---|
| H,H-COSY: H6a-H7, H6e-H7, H7-H29 (w) = weak | | | |

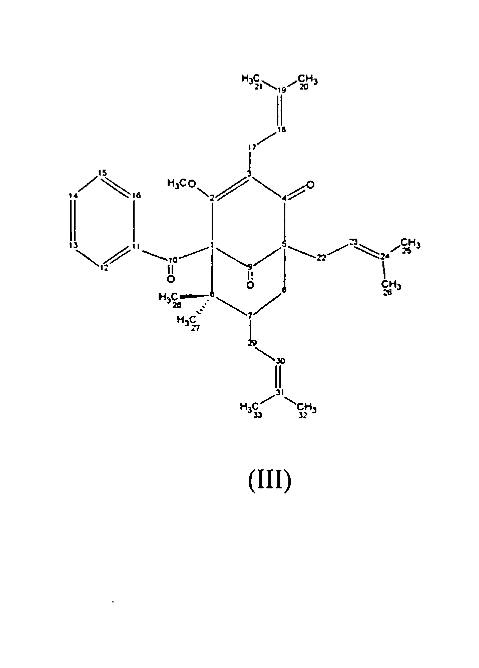

### Absolutkonfiguration

Aufgrund der zuvor aufgeführten NMR-Daten konnte der Verbindung der allgemeinen Formel (Ic) bzw. (Ic') bzw. (Ic") die folgende Absolutkonfiguration zugeordnet werden:

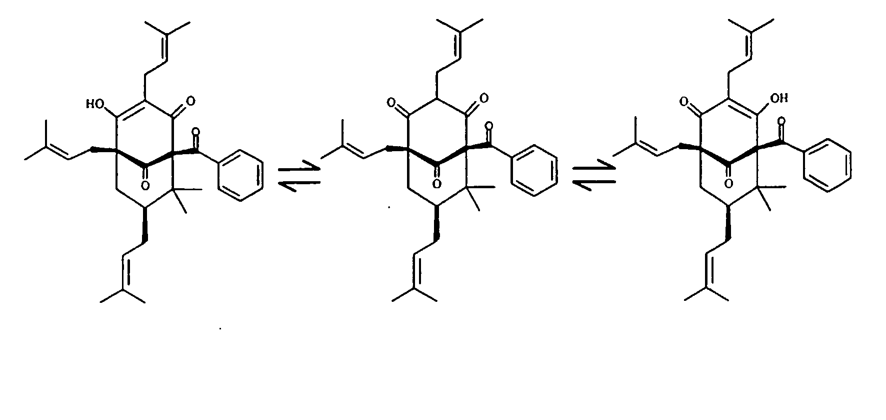

### Zellkulturen

Sechs eindeutig charakterisierte tumorale Zell-Linien humanen Ursprungs (native wie auch in resistente Subklone überführte Zell-Linien) sowie zwei nichttumorale Zell-Linien werden für weitere Untersuchungen herangezogen (Tabelle 2).

**Tabelle 2: Zell-Linien, histologische Charakterisierung der Zell-Linien und ihre Herkunft**

| **Zell-Line** | **Histologische Charakterisierung** | **RF** | **Quelle** |
|---|---|---|---|
| **M 51** | Magenkarzinom | | Hannover |
| **M51 DDP** | Cisplatin-resistent | 8,5 | Medizinische Fakultät |
| **HT29 WT** | Kolonadenokarzinom | | ATCC HTB 38 |
| **HT29 24R** | 5-FU-resistent | 3,7 | Universität Essen |
| **HT29 SN38** | SN38-resistent | 3,6 | Medizinische Fakultät |
| **HCT8 WT** | Kolonkarzinom | | ATCC CCL24 |
| **HCT8 SN38** | SN38-resistent | 6,9 | Universität Essen |
| **HCT8 ICID** | Raltitrexed-resistent | 2,9 | Medizinische Fakultät |
| **MCF-7 WT** | Brustadenokarzinom | | ATCC HTB 22 |
| **MCF-7 AD** | Doxorubicin-resistent gp 170+ | 222 | Roswell Park Cancer |
| **MCF-7 24R** | 5-FU-resistent | 6,5 | Institute |
| **A2780 WT** | Eierstockadenokarzinom | | Roswell Park Cancer |
| **A2780 DX5** | Doxorubicin-resistent gp 170+ | 15,6 | |
| **A2780 CP2** | Cisplatin-resistent | 15,0 | Institute |
| **H460 WT** | Großzelliges Lungenkarzinom | | ATCC HTB 117 |
| **3T3** | Mäusefibroblasten | | ATCC CCL 163 |
| **MCR-5** | Diploide embryonale Lungenfibroblasten | | ATCC CCL 171 |

### SRB-Test

Die Sensitivität der Zell-Linien gegenüber der Substanz (Ic') wird in einem sogenannten Sulphorhodamin B (SRB)-Test ermittelt. Hierbei handelt es sich um ein robustes und hoch reproduzierbares Standardverfahren, wie es auch z. B. am NCI (National Cancer Institute, USA) etabliert ist (Skehan et al. 1990).

Die Ergebnisse der Tests sind in der Tabelle 3 wiedergegeben. Die Konzentration an Substanz (Ic'), welche das Zellwachstum um 50 % inhibiert (IC₅₀, ermittelt aus halblogarithmischer Dosis gegen Wirkungskurven) sind angegeben. Als Ergebnis werden in der Tabelle 3 Mittelwerte und ihre Standardabweichungen von mindestens drei unabhängigen Experimenten dargestellt. Es gibt keine Kreuzresistenz zu den angegebenen Cytostatika. Gegenüber den nichttumoralen Zell-Linien (Fibroblasten der 3T3- und MCR 5- Linien) erweist sich die Substanz (Ic') als wesentlich weniger cytotoxisch.

**Tabelle 3: Ermittelte IC₅₀-Konzentrationen an Substanz (Ic') in den unterschiedlichen Zell-Linien.**

| | **Substanz (Ic')** | |
|---|---|---|
| **Zell-Linie** | **IC** _{**50**} **(µg/ml)** | |
| **M51 WT** | 5,70 ± 0,21 | |
| **M51 DDP** | 4,77 ± 0,13 | |
| **HT29 WT** | 5,25 ± 0,43 | |
| **HT2924R** | 5,18 ± 0,76 | |
| **HT29 SN38** | 3,50 ± 1,40 | |
| **HCT8 WT** | 4,23 ± 0,19 | |
| **HCT8 SN38** | 4,07 ± 0,08 | |
| **HCT8 ICID** | 4,44 ± 0,13 | |
| **MCF-7 WT** | 4,37 ± 0,12 | |
| **MCF-7 AD** | 4,28 ± 0,08 | |
| **MCF-7 24R** | 3,30 ± 0,51 | |
| **A2780 WT** | 9,20 ± 0,82 | |
| **A2780 DX5** | 6,75 ± 1,55 | |
| **A2780 CP2** | 6,23 ± 0,12 | |
| **H460 WT** | 4,21 ± 0,91 | |
| **3T3** | > 80, 0 | |
| **MCR-5** | 21,73 ± 6,47 | |

### Untersuchungen zur Inhibition der Topoisomerase I

Um den Effekt der Substanz (Ic') auf die Topoisomerase I zu untersuchen, wird der sogenannte "Topo I unwinding" Test durchgeführt. Der Test beruht auf einer ATP-unabhängigen "unwinding" (Entknäuelung) von verdrillter pBR322-Plasmid-DNA-Dimeren durch die Topoisomerase I. Die beiden verschiedenen topologischen Formen der Plasmid-DNA, die verdrillte Form und die entknäuelte, relaxierte Form, werden bei dieser Untersuchung mittels eines Agarosegels getrennt. Das Enzym Topoisomerase I wird dazu aus Zellkernextrakten der tumoralen Zell-Linien gewonnen.

### Gewinnung der Zellkernextrakte

Zellkernextrakte werden entsprechend der Methode von Sullivan mit den Modifikationen von Danks et al. gewonnen.

### Inhibition der Topoisomerase I-Aktivität durch Substanz (Ic')

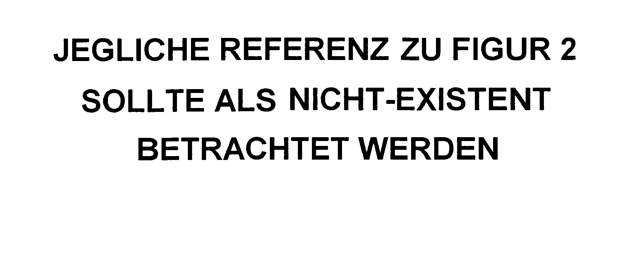

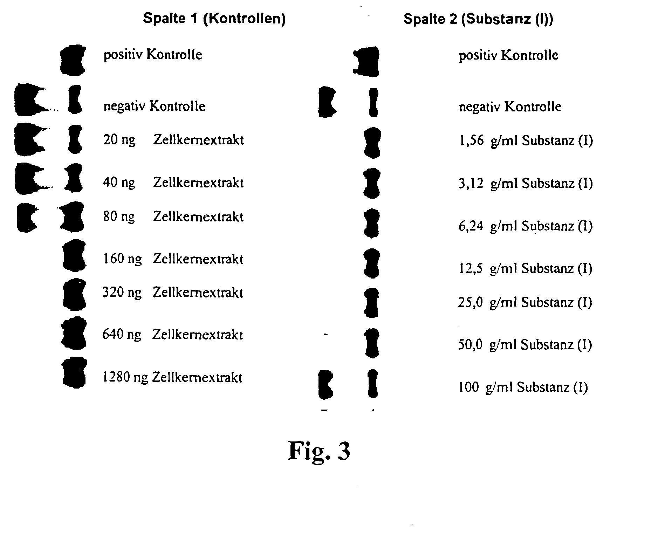

Wie in Fig. 3 (Spalte 1: Kontrollen) gezeigt, werden 160 ng Zellkernextrakt mit der darin enthaltenen Topoisomerase I benötigt, um die hochverdrillten pBR322-Plasmid-DNA-Dimeren (Negativkontrolle) in die relaxierte Topologie (Positivkontrolle) zu überführen. Die Substanz (Ic') inhibiert die Topoisomerase I-Aktivität zu 100 % bei einer Konzentration von 100 µg/ml. Dies entspricht einem 20fachen der benötigten Konzentration, die das tumorale Wachstum der korrespondierenden Zell-Linien zu 50 % inhibiert (IC₅₀, Fig. 2). Vergleicht man diese Daten mit der in-vitro-inhibitorischen Aktivität von z. B. Topotecan, so wird für diese bereits in der Klinik etablierte Substanz, das 1000fache der IC₅₀ benötigt. Fig. 3 veranschaulicht die Inhibition der Topoisomerase I-Aktivität durch Substanz (Ic').

### Zellzyklusanalysen

Die bisher beschriebenen Wirkungen der Substanz (Ic') auf das Zellwachstum lassen sich auch mit Veränderungen im Zellzyklus belegen. Durchflußcytometrische Untersuchungen nach einer angepaßten Methode von Tsugita M. et al. werden zur Analyse herangezogen.
Exponentiell wachsende HCT8 WT-Darmkrebszellen werden mit der Substanz (Ic') mit der ermittelten IC₅₀-Konzentration für 24 Stunden behandelt, danach werden die Zellen mit BrdU inkubiert. Die mit Methanol fixierten Zellen werden einer Behandlung mit einem Detergens (Triton X-100/HCl) unterworfen, um die DNA-Stränge zu denaturieren. Nach Inkubation mit dem fluoreszenzmarkierten Antikörper (anti-BrdU mAb, FITC-markiert) wird der zweite Fluoreszenzfarbstoff (PI, Propidiumiodid) hinzugegeben.

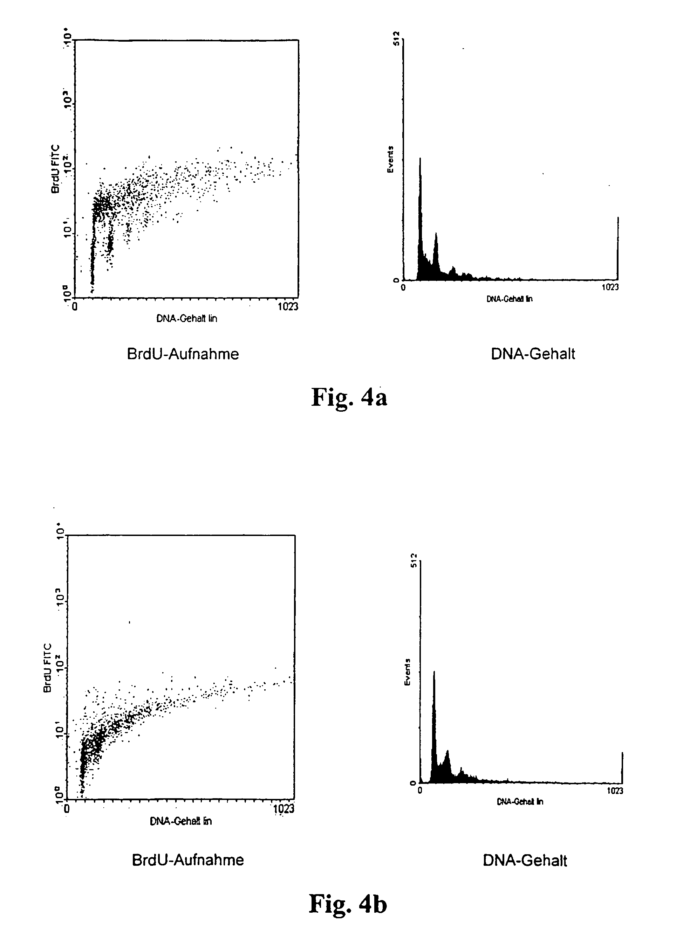

Der Gehalt an DNA (Propidiumiodid-Fluoreszenz) und die Menge an aufgenommenem BrdU (FITC-Fluoreszenz) werden mittels eines Durchflußcytometers der Firma Coulter bestimmt (Coulter EPICS XL). Beispielhafte Ergebnisse sind in Fig. 4a und 4b dargestellt.

Die Fig. 4a und 4b zeigen BrdU- und PI-Aufnahmen in die DNA von HCT8 WT-Zellen, nicht behandelt (Kontrolle, Fig. 4a) und mit Substanz (Ic') behandelt (IC₅₀, 24 Stunden, Fig. 4b). Die dargestellten Ergebnisse sind repräsentativ für n = 3 unabhängige Experimente.

Offensichtlich führt die Behandlung mit Substanz (Ic') zu einer Inhibition der Synthese-Phase. Dies wird anhand der Abbildungen zum DNA-Gehalt (Abb. 4a und 4b, BrdU-Aufnahme) deutlich.

### Analyse der DNA-Degradation

Die Untersuchungen zum Nachweis der Degradation der DNA durch die Substanz (Ic') werden beispielhaft an humanen Zellen eines Bronchialkarzinoms (H460 WT) und eines kolorektalen Karzinoms (HT29 WT) durchgeführt. DNA, isoliert nach Behandlung der Zellen für unterschiedliche Dauer mit der Substanz (Ic'), wird dazu gelelektrophoretisch analysiert.

Die Bestimmung der Doppelstrangbrüche der DNA gelingt an ¹⁴C-markierter DNA. Dazu werden [¹⁴C]d-Thymidin gelabelte Zellen mit unterschiedlichen Konzentrationen an Substanz (Ic') behandelt. Die Isolierung der DNA erfolgt nach einer angepaßten Methode von Chia Chiao et al.

Nach Bestimmung der Menge und der Reinheit der isolierten DNA mittels eines GeneQuant Spektrophotometers werden vergleichbare Mengen an DNA mittels eines Agarosegels aufgetrennt. Die gelelektrophoretisch aufgetrennte DNA wird anschließend mit Ethidiumbromid angefärbt.

Auf einem Tansilluminator (302 nm) wird die nicht im elektrischen Feld gewanderte intakte DNA von der fragmentierten und damit in das Gel eingewanderten DNA durch Extraktion getrennt. Die isolierten Gel- bzw. DNA-Fragmente werden bei + 80 °C in Anwesenheit von Salzsäure verflüssigt. Nach Zugabe eines Szintillators kann die Radioaktivität mittels eines Flüssigszintillationsmeßgerätes (TPI-CARB 2100RT) bestimmt werden. Die gemessene Radioaktivität wird in Zerfälle pro Minute (cpm) angegeben.

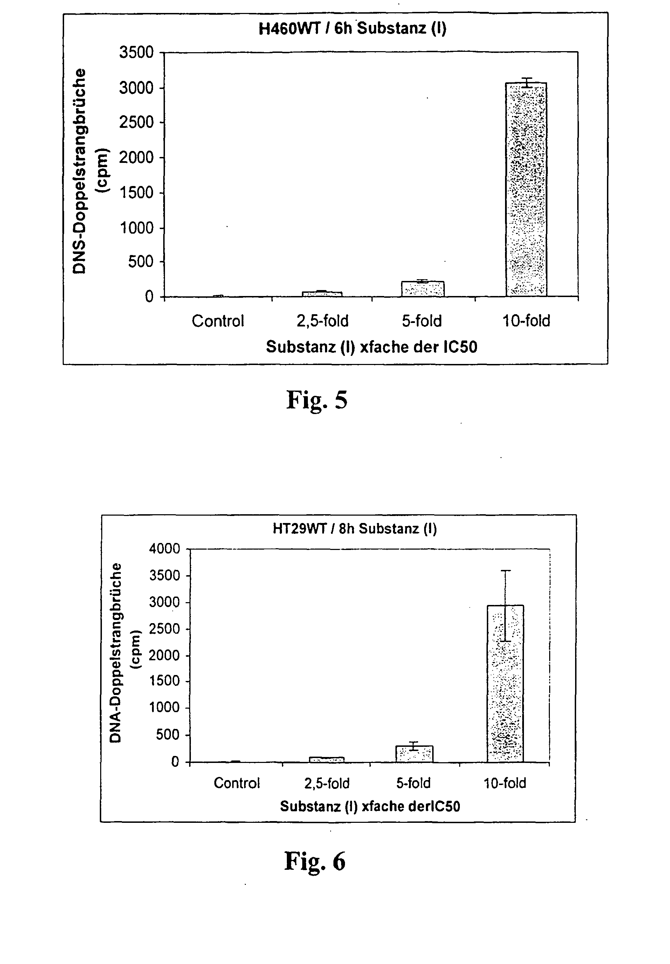

In Fig. 5 ist die Induktion der Doppelstrangbrüche durch die Substanz (Ic') nach Inkubation von bronchialen Karzinomzellen (H480 WT) für 6 Stunden gezeigt. Fig. 5 zeigt DNA-Doppelstrangbrüche, nachgewiesen in [¹⁴C]d-Thygelabelten H460 WT- Zellen, ausgelöst durch die Behandlung mit Substanz (Ic') für 6 Stunden. Die Ergebnisse stellen Mittelwerte und ihre Standardabweichung aus n = 3 unabhängigen Experimenten dar.

Durch die Behandlung der Tumorzellen mit bis zu einem 10fachen der ermittelten IC₅₀-Konzentration werden innerhalb von 6 Stunden signifikante Mengen an Doppelstrangbrüchen induziert, welche offensichtlich schon bei der IC₅₀-Konzentration nicht mehr ausreichend durch die Tumorzellen repariert werden und somit den Zelltod einleiten.

In Fig. 6 ist ein weiteres Beispiel der Induktion von Doppelstrangbrüchen in der DNA von Tumorzellen, hier Zellen eines kolorektalen Tumors (HT29 WT), aufgezeigt. Wie schon bei der Zell-Linie des Bronchialkarzinoms, werden offensichtlich substantielle Mengen an Doppelstrangbrüchen induziert, welche nicht wieder durch die Reparaturmechanismen der Tumorzelle korrigiert werden können und letztendlich mit ein auslösender Faktor für die Apoptose, den programmierten Zelltod der Tumorzelle darstellt.

### Bestimmung der inhibitorischen Aktivität von Substanz (Ic') auf die humane Telomerase

Um die Wirkung der Substanz (Ic') auf die humane Telomerase zu bestimmen wird der sogenannte "Telomeric Repeat Amplification Assay" (TRAP)-ELISA, basierend auf der Methode von N. W. Kim et al., durchgeführt.
Diese Methode basiert auf der Nutzung der Polymerasen-Kettenreaktion (PCR). Dabei wird in einem ersten Schritt mittels der Telomerase-Aktivität eine Anzahl von (GGTTAG)-Motiven an das 3'-Ende eines mittels Biotin markierten Oligonukleotids (TS = Telomerase-Substrat) angehängt. In einem zweiten Schritt werden die so verlängerten Produkte mittels der PCR-Technik unter Nutzung von TS- und RP- (R = reverse) Primern vervielfältigt. Auf diese Art und Weise wird eine "Leiter" von Produkten in Inkrementen zu 6 Basen (Startgröße = 50 Nukleotide) generiert. Während der Vervielfältigung wird DNP-markiertes dCTP in die PCR-Produkte eingebaut. Die Detektion und Quantifizierung der synthetisierten Telomeraseprodukte mittels einer "Sandwich ELISA"-Methode sind ein Maß für die enzymatische Aktivität der Telomerase.

Die Produkte der Telomerase tragen die mittels Biotin markierten TS-Primer und binden an der Oberfläche von Mikrotiterplatten, welche vorher mit Streptavidin beschichtet werden. Ein anti-DIG Antikörper, konjugiert mit POD, bindet an die während der Vervielfältigung eingebauten DNP-markierten Nukleotide. Nach Zugabe des Substrates TMB wird über die einsetzende Farbreaktion durch das Enzym POD die Aktivität der Telomerase fluoreszenzspektroskopisch sichtbar gemacht. Dabei ist die gemessene Absorption direkt proportional zur Aktivität der Telomerase.

### Effekt der Substanz (Ic') auf die Aktivität humaner Telomerase

Um die Wirkung der Substanz (Ic') auf die Telomerase sicherzustellen, muß eine Wirkung der Substanz (Ic') auf die DNA-Polymerase ausgeschlossen werden. In einem Vorexperiment mit TSR8 als interne Kontrolle wurde die Wirkung von Substanz (Ic') bei verschiedenen Konzentrationsstufen ausgeschlossen.

Die Vervielfältigung des TSR8 Templates ist nur von der DNA-Polymerase abhängig. Jeder Inhibitor dieses Enzyms kann mit diesem Substrat nachgewiesen werden.

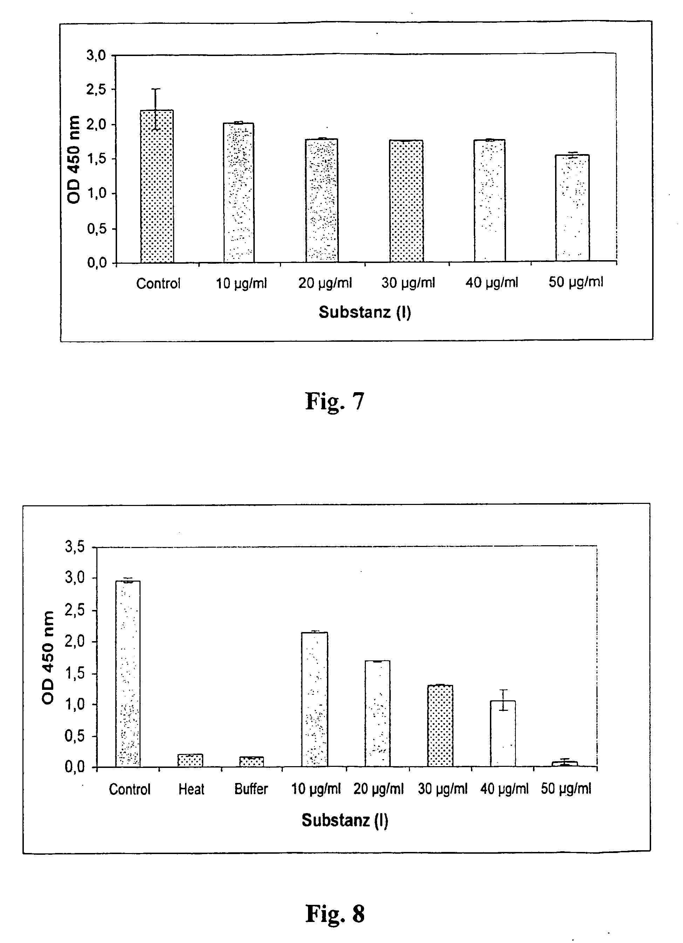

Fig. 7 zeigt die Ergebnisse des Vorexperiments mit dem TSR8-Template. Das Experiment wird in Triplikaten durchgeführt. Fig. 7 belegt, daß selbst die maximal eingesetzte Konzentration von 50 µg/ml der Substanz (Ic') in diesem Experiment nicht zu einer signifikanten Inhibition der Taq-Polymerase führt.

Das Experiment zur Untersuchung einer Inhibition der Telomerase durch die Substanz (Ic') wird in Fig. 8 gezeigt. Dargestellt ist der Konzentrationsbereich zwischen 10 und 50 µg/ml. Eine komplette Inhibition humaner Telomerase wird bei einer Konzentration von 50 µg/ml beobachtet. Fig. 8 zeigt die Ergebnisse des TRAP-Assays: Dosisabhängige Inhibition humaner Telomerase, gewonnen aus Zellen eines Kolonkarzinoms (HCT8 WT), durch die Substanz (Ic'). Die Ergebnisse stellen Mittelwerte und Standardabweichungen dreier unabhängiger Experimente dar.

### Phosphorylierung von ERK1/2 (MAP-Kinase-Pathway)

Die Phosphorylierung bestimmter Signaltransduktionswege spielt eine essentielle Rolle bei der Regulation von Zellwachstum bzw. Genexpression. Das Protein ERK1/2 (MAP-Kinase) gehört zu dem MAP-Kinase-Signaltransduktionsweg.

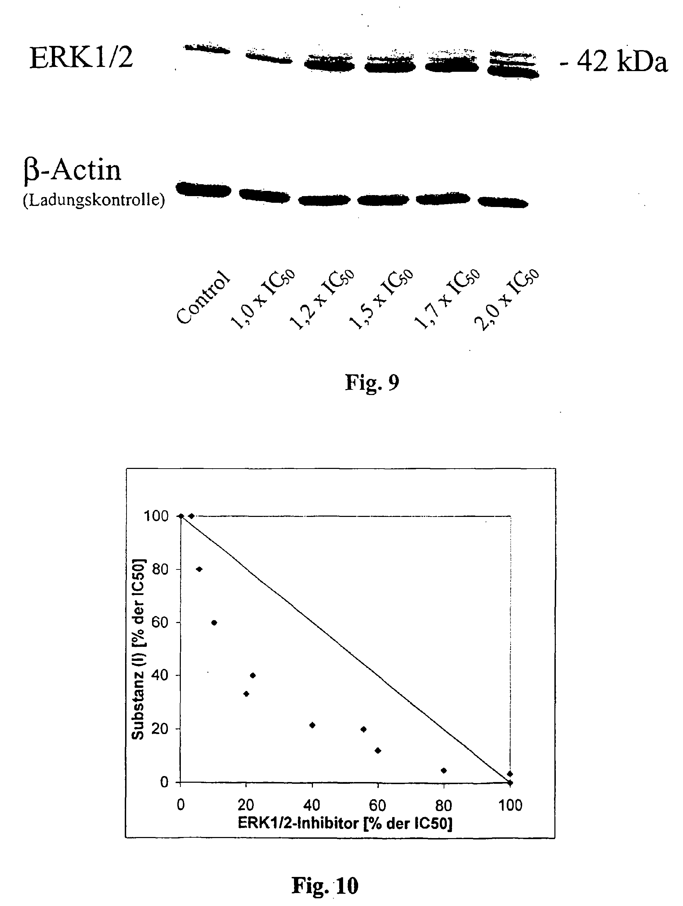

Die Behandlung humaner Kolonkarzinomzellen (HCT8 WT) mit der Substanz (Ic') führt zu einer Phosphorylierung dieses Proteins, welches in einer Western-Blot-Analyse nachgewiesen wird (Fig. 9). Fig. 9 zeigt die Westem-Blot-Analyse des ERK1/2-Proteins; in der unteren Zeile ist die Ladungskontrolle mitgeführt.

Die Behandlung der Zellen mit der Substanz (Ic') führt dosisabhängig zu einer Phosphorylierung des ERK1/2-Proteins. Schon ab einer Dosis der IC₅₀ entsprechend läßt sich die Phosphorylierung durch die Substanz (Ic') belegen. Als Resultat dieses - bisher in der Literatur als Aktivierung gedeuteten - Prozesses wird die Tumorzelle in einen Zellzyklusarrest geschickt, was durch die durchgeführten Zellzyklusanalysen belegt wird (s. o.).

Zur Überprüfung dieser Befunde, inwieweit die erzielten Ergebnisse hinsichtlich einer ERK1/2-Aktivierung durch die Substanz (Ic') durch Zugabe eines ERK1/2-Inhibitors antagonisierbar sind, wird ein Koinkubationsexperiment mit der Substanz (Ic') und einem spezifischen ERK1/2-Inhibitor (MAP-Kinase-Inhibitor) durchgeführt. Dabei werden Tumorzellen, z.B. Kolonkarzinomzellen (HCT8 WT), mit beiden Wirkstoffen in jeweils unterschiedlichen Dosierungen für 24 Stunden koinkubiert (Fig. 10). Fig. 10 gibt das Koinkubationsexperiment zur Cytotoxizität mit der Substanz (Ic') und einem spezifischen ERK1/2-Inhibitor (MAP-Kinase-Inhibitor) wieder. Koinkubiert wurden HCT8 WT-Zellen. Die gezeigten Punkte geben die entsprechenden Verhältnisse (% der jeweiligen IC₅₀-Konzentration) beider Wirkstoffe zueinander an.

Dieses derart durchgeführte Experiment führt aber in keinem Dosierungsverhältnis zu einer Aufhebung der gemessenen Cytotoxizität. Vielmehr ist ein ausgeprägter Synergismus bei der Koinkubation beider Wirkstoffe zu verzeichnen.

Alle Dosierungskombinationen liegen unterhalb der Winkelhalbierenden, und damit im Bereich eines eindeutigen Synergismus. Die Kombination von 30 % der IC₅₀-Konzentration von Substanz (Ic') mit einer Wirkstoffkonzentration von 30 % der IC₅₀-Konzentration des spezifischen ERK1/2-Inhibitors führt zu einer Wachstumsinhibition der Tumorzellen, isoliert aus einem Kolonkarzinom (HCT8 WT).

## Patentansprüche

1. Verwendung substituierter Bicyclo[3.3.1]nonan-2,4,9-trione der allgemeinen Formel (I) 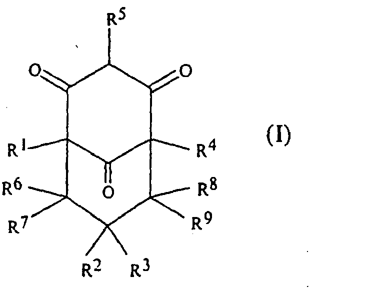 in der:
• R¹, R², R⁴ und R⁵, gleich oder verschieden, unabhängig voneinander eine der folgenden Gruppen darstellen: 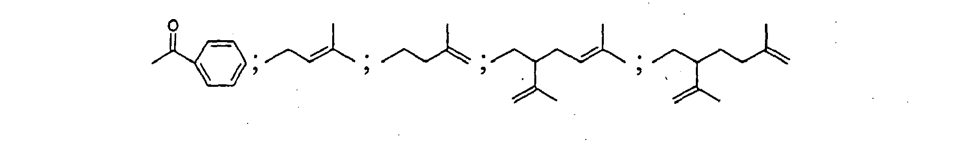 jedoch mit der Maßgabe, daß, wenn einer der Reste R¹ oder R² oder R⁴ oder R⁵ eine Benzoylgruppe darstellt, keiner der anderen dieser Reste eine Benzoylgruppe darstellt;
• R³ ein Wasserstoffatom darstellt;
• R⁶ und R⁷ beide jeweils eine Methylgruppe oder aber jeweils ein Wasserstoffatom darstellen und R⁸ und R⁹ beide jeweils eine Methylgruppe oder aber jeweils ein Wasserstoffatom darstellen, jedoch mit der Maßgabe, daß, wenn R⁶ und R⁷ Methylgruppen darstellen, R⁸ und R⁹ Wasserstoffatome sind und daß, wenn R⁸ und R⁹ Methylgruppen darstellen, R⁶ und R⁷ Wasserstoffatome sind;
und ihrer physiologisch verträglichen Salze, Hydrate, Isomeren, insbesondere Stereoisomeren, Tautomeren und Konstitutionsisomeren, Derivate, zur Herstellung von Arzneimitteln zur prophylaktischen und/oder therapeutischen Behandlung von Tumor- und/oder Krebserkrankungen.

2. Verwendung nach Anspruch 1, **dadurch gekennzeichnet, daß** die Verbindungen der Formel (I) ausgewählt sind aus der Gruppe der folgenden Verbindungen:

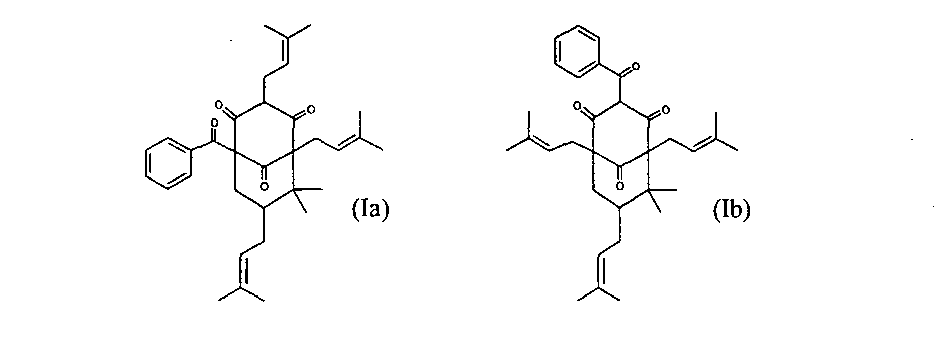

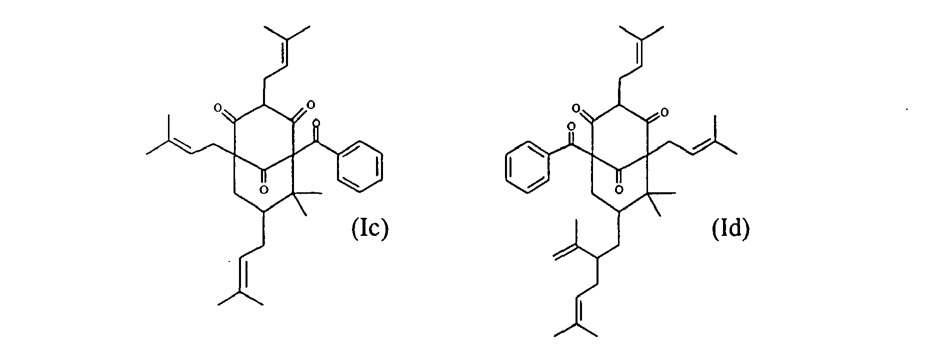

3. Verwendung nach Anspruch 1 oder 2, **dadurch gekennzeichnet, daß** die Verbindung der Formel (I) die folgende Verbindung der Formel (Ic) ist:

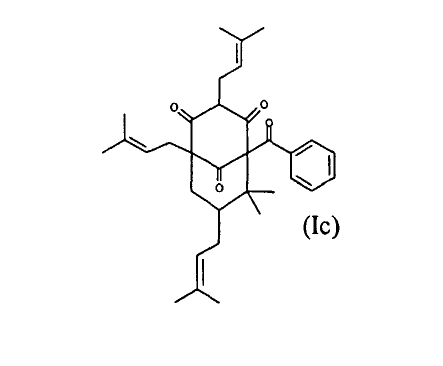

4. Verwendung nach Anspruch 3, **dadurch gekennzeichnet, daß** die Verbindung der Formel (Ic) die folgende Konfiguration aufweist:

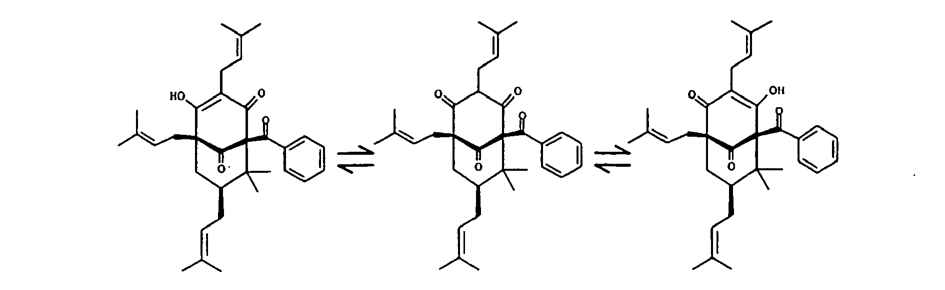

5. Verwendung nach einem der Ansprüche 1 bis 4 zur prophylaktischen und/oder therapeutischen Behandlung von Tumor- und/oder Krebserkrankungen, insbesondere Primärtumoren und Metastasen, und/oder Präkanzerosen (Krebsvorstufen).

6. Verwendung nach einem der Ansprüche 1 bis 5 zur prophylaktischen und/oder therapeutischen Behandlung benigner und maligner Tumore.

7. Verwendung nach einem der Ansprüche 1 bis 6 zur prophylaktischen und/oder therapeutischen Behandlung von Darmkrebs (Kolonkarzinomen), Brustkrebs (Mammakarzinomen), Ovarialkarzinomen, Uteruskarzinomen, Lungenkrebs, Magenkrebs, Leberkrebs, Pankreaskarzinomen, Nierenkrebs, Blasenkrebs, Prostatakrebs, Hodenkrebs, Knochenkrebs, Hautkrebs, Kaposi-Sarkomen, Hirntumoren, Myosarkomen, Neuroblastomen, Lymphomen und Leukämien.

8. Verwendung nach einem der Ansprüche 1 bis 7, **dadurch gekennzeichnet, daß** die Verbindungen der allgemeinen Formel (I) systemisch und/oder topisch appliziert werden.

9. Verwendung nach einem der Ansprüche 1 bis 8, **dadurch gekennzeichnet, daß** die Verbindungen der allgemeinen Formel (I) das Zellwachstum und die Zellteilung von Tumor- und/oder Krebszellen hemmen und/oder zum Stillstand bringen.

10. Verwendung nach einem der Ansprüche 1 bis 9, **dadurch gekennzeichnet, dass** die Verbindungen der allgemeinen Formel (I) eine Zerstörung der DANN der Tumor- und/oder Krebszellen induzieren.

11. Verwendung von Verbindungen der allgemeinen Formel (I), wie zuvor definiert, und/oder ihrer physiologisch verträglichen Salze, Hydrate, Isomeren, insbesondere Stereoisomeren, Tautomeren und Konstitutionsisomeren, Derivate, Prodrugs und Metabolite zur Herstellung von Topoisomeraseinhibitoren und/oder Telomeraseinhibitoren.

12. Verwendung nach einem der Ansprüche 1 bis 11, **dadurch gekennzeichnet, dass** die Verbindungen der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite in Kombination mit mindestens einem weiteren Chemotherapeutikum zur Herstellung eines Arzneimittels, insbesondere Proteinkinaseinhibitors, vorzugsweise MAP-Kinase-Inhibitors, verwendet werden.

13. Cytostatikum, enthaltend mindestens eine Verbindung der allgemeinen Formel (I), wie zuvor definiert, und/oder ihre physiologisch verträglichen Salze, Hydrate, Isomere, insbeosndere Stereoisomere, Tautomere und Kosntitutionsisomere, Derivate, Prodrugs und Metabolite zusammen mit einem pharmazeutisch verträglichen, im wesentlichen nichttoxischen Träger oder Exzipienten.

14. Cytostaticum nach Anspruch 13, enthaltend die mindestens eine Verbindung der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite in therapeutisch wirksamen Mengen.

15. Pharmazeutische Zusammensetzung oder Arzneimittel nach Anspruch 13 oder 14, zur systemischen und/oder topischen Anwendung.

16. Pharmazeutische Kombination, insbesondere cytostatische Kombination, die
(A) mindestens eine Verbindung der allgemeinen Formel (I), wie zuvor definiert, und/oder ihre physiologisch verträglichen Salze, Hydrate, Isomere, insbesondere Stereoisomere, Tautomere und Kosntitutionsisomere, Derivate, Prodrugs und Metabolite; und
(B) mindestens ein weiteres Chemotherapeutikum, insbeosndere einen Proteinkinaseinhibitor, vorzugsweise MAP-Kinase-Inhibitor.
umfaßt.

17. Pharmazeutische Kombination nach Anspruch 16, **dadurch gekennzeichnet, dass** die Komponenten (A) und (B) entweder als funktionelle Einheit, insbesondere in Form einer Mischung, eines Gemisches oder eines Gemenges, oder aber (räumlich) getrennt voneinander vorliegen.

18. Pharmazeutische Kombination nach Anspruch 16 oder 17, wobei die Komponenten (A) und (B) entweder gleichzeitig oder aber zeitlich abgestuft anwendbar oder applizierbar sind.

19. Pharmazeutische Kombination nach einem der Ansprüche 16 bis 18, enthaltend die Komponenten (A) und (B) jeweils in therapeutisch wirksamen Mengen.

20. Pharmazeutische Kombination nach einem der Ansprüche 16 bis 19, zur systemischen und/oder topischen Anwendung.

21. Verbindungen der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite zur prophylaktischen und/oder therapeutischen Behandlung von Krebserkrankungen, insbesondere Primärtumoren und Metastasen, und/oder Präkanzerosen (Krebsvontufen), insbesondere von Darmkrebs (Kolonkarzinumen), Brustkrebs (Mammakarzinomen), Ovarialkarzinomen, Uteruskarzinomen, Lungenkrebs, Magenkrebs, Leberkrebs, Pankreaskarzinomen, Nierenkrebs, Blasenkrebs, Prostatakrebs, Hodenkrebs, Knochenkrebs, Hautkrebs, Kaposi-Sarkomen, Hirntumoren, Myosarkomen, Neuroblastomen, Lymphomen und Leukämien.

22. Verbindungen der allgemeinen Formel (I), wie zuvor definiert, und/oder ihre physiologisch verträglichen Salze, Hydrate, Isomere, insbesondere Stereoisomere, Tautomere und Konstitutiunsisomere, Derivate, Prodrugs und Metabolite zur Inhibierung von Topoisomerasen und/oder Telomerasen.

23. Verbindungen der allgemeinen Formel (I), wie zuvor definiert, und/oder ihre physiologisch verträglichen Salze, Hydrate, Isomere, insbesondere Stereoisomere, Tautomere und Konstitutionsisomere, Derivate, Prodrugs und Metabolite, als Regulatoren innerhalb der MAP-Kinase-Signaltransduktion.

## Claims

1. Use of substituted bicyclo[3.3.1]nonane-2,4,9-triones of the general formula (I) 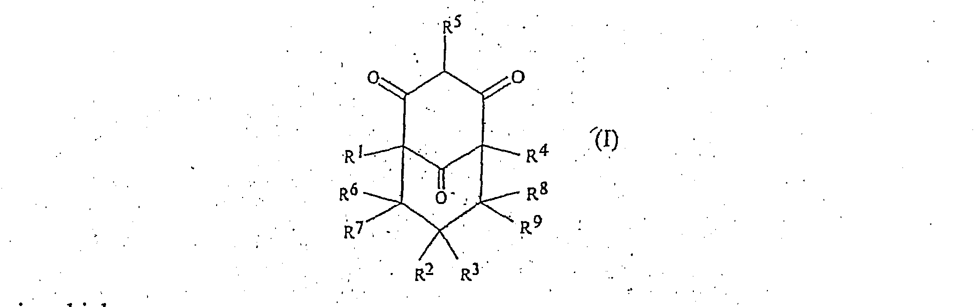 in which:
• R¹, R², R⁴ and R⁵ are identical or different, are independent of one another and represent one of the following groups: 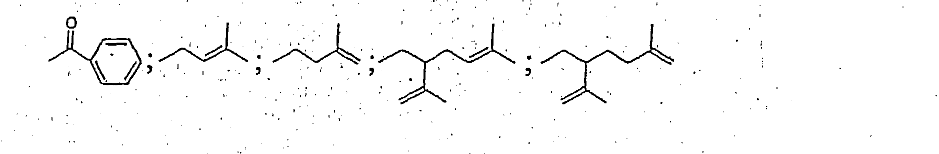 with the proviso, however, that, when one of the radicals R¹ or R² or R⁴ or R⁵ represents a benzoyl group, none of these other radicals represents a benzoyl group;
• R³ represents a hydrogen atom;
• R⁶ and R⁷ both represent respectively a methyl group or respectively, however, a hydrogen atom, and R⁸ and R⁹ both represent respectively a methyl group or respectively, however, a hydrogen atom, with the proviso, however, that, when R⁶ and R⁷ represent methyl groups, R⁸ and R⁹ are hydrogen atoms, and that, when R⁸ and R⁹ represent methyl groups, R⁶ and R⁷ are hydrogen atoms;
and use of their physiologically compatible salts, hydrates, isomers, more especially stereoisomers, tautomers and constitutional isomers and derivatives to produce drugs for the prophylactic and/or therapeutic treatment of tumour-related and/or cancerous diseases.

2. Use according to claim 1, **characterised in that** the compounds of Formula (I) are selected from the group of the following compounds:

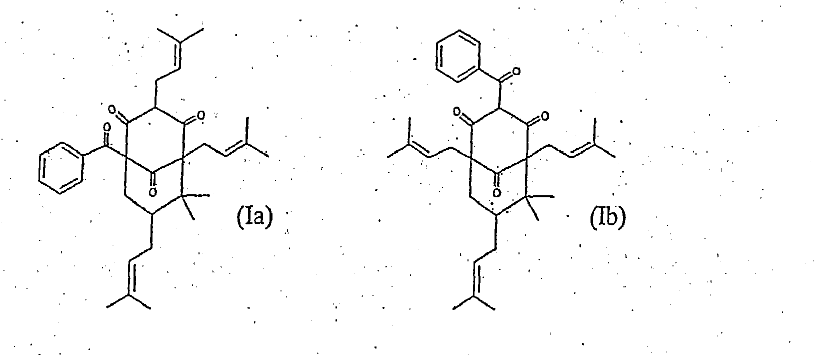

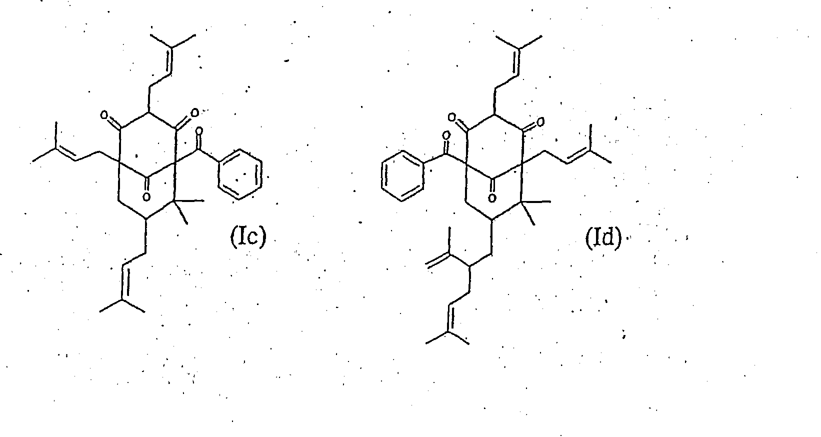

3. Use according to claim 1 or 2, **characterised in that** the compound of Formula (I) is the following compound of Formula (Ic):

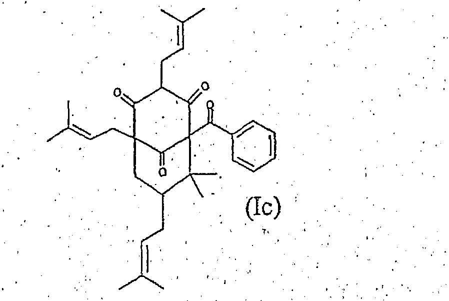

4. Use according to claim 3, **characterised in that** the compound of Formula (Ic) has the following configuration:

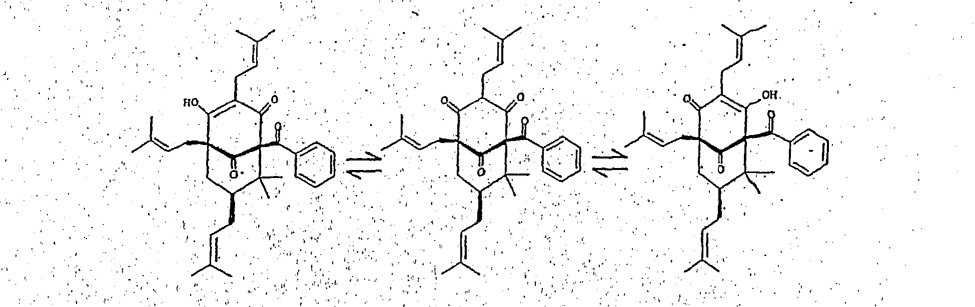

5. Use according to one of claims 1 to 4 for the prophylactic and/or therapeutic treatment of tumour-related and/or cancerous diseases, more especially primary tumours and metastases, and or precanceroses (precancerous stages).

6. Use according to one of claims 1 to 5 for the prophylactic and/or therapeutic treatment of benign and malignant tumours.

7. Use according to one of claims 1 to 6 for the prophylactic and/or therapeutic treatment of bowel cancer (colon carcinomas), breast cancer (mammary carcinomas), ovarian carcinomas, uterine carcinomas, lung cancer, stomach cancer, liver cancer, pancreatic carcinomas, renal cancer, bladder cancer, prostate cancer, testicular cancer, bone cancer, skin cancer, Kaposi sarcomas, brain tumours, myosarcomas, neuroblastomas, lymphomas and leukaemias.

8. Use according to one of claims 1 to 7, **characterised in that** the compounds of the general formula (I) are applied systemically and/or topically.

9. Use according to one of claims 1 to 8, **characterised in that** the compounds of the general formula (I) check and/or arrest the cellular growth and the cellular division of tumour cells and/or cancer cells.

10. Use according to one of claims 1 to 9, **characterised in that** the compounds of the general formula (I) induce a destruction of the DANN of the tumour cells and/or cancer cells.

11. Use of compounds of the general formula (I), as defined previously, and/or of their physiologically compatible salts, hydrates, isomers, more especially stereoisomers, tautomers and constitutional isomers, derivatives, prodrugs and metabolites for the production of topoisomerasis inhibitors and/or telomerasis inhibitors.

12. Use according to one of claims 1 to 11, **characterised in that** the compounds of the general formula (I), as defined previously, and/or their physiologically compatible salts, hydrates, isomers, more especially stereoisomers, tautomers and constitutional isomers, derivatives, prodrugs and metabolites are used in combination with at least one additional chemotherapeutic agent for producing a drug, more especially a protein kinasis inhibitor, preferably a MAP kinasis inhibitor.

13. Cytostatic agent, containing at least one compound of the general formula (I), as defined previously, and/or its physiologically compatible salts, hydrates, isomers, more especially stereoisomers, tautomers and constitutional isomers, derivatives, prodrugs and metabolites together with a pharmaceutically compatible, substantially non-toxic carrier or excipient.

14. Cytostatic agent according to claim 13, containing the at least one compound of the general formula (I), as defined previously, and/or its physiologically compatible salts, hydrates, isomers, more especially stereoisomers, tautomers and constitutional isomers, derivatives, prodrugs and metabolites in therapeutically effective quantities.

15. Pharmaceutical composition or drug according to claim 13 or 14, for systemic and/or topical application.

16. Pharmaceutical combination, more especially a cytostatic combination, which includes
(A) at least one compound of the general formula (I), as defined previously, and/or its physiologically compatible salts, hydrates, isomers, more especially stereoisomers, tautomers and constitutional isomers, derivatives, prodrugs and metabolites; and
(B) at least one additional chemotherapeutic agent, more especially a protein kinasis inhibitor, preferably a MAP kinasis inhibitor.

17. Pharmaceutical combination according to claim 16, **characterised in that** the components (A) and (B) are either present as a functional unit, more especially in the form of a mix, a mixture or a batch, or, however, are (spatially) separated from each other.

18. Pharmaceutical combination according to claim 16 or 17, wherein the components (A) and (B) are able to be used or applied either simultaneously or at timed intervals.

19. Pharmaceutical combination according to one of claims 16 to 18, containing the components (A) and (B) each in therapeutically effective quantities.

20. Pharmaceutical combination according to one of claims 16 to 19, for systemic and/or topical application.

21. Compounds of the general formula (I), as defined previously, and/or their physiologically compatible salts, hydrates, isomers, more especially stereoisomers, tautomers and constitutional isomers, derivatives, prodrugs and metabolites for the prophylactic and/or therapeutic treatment of cancerous diseases, more especially primary tumours and metastases, and/or precanceroses (precancerous stages), more especially of bowel cancer (colon carcinomas), breast cancer (mammary carcinomas), ovarian carcinomas, uterine carcinomas, lung cancer, stomach cancer, liver cancer, pancreatic carcinomas, renal cancer, bladder cancer, prostate cancer, testicular cancer, bone cancer, skin cancer, Kaposi sarcomas, brain tumours, myosarcomas, neuroblastomas, lymphomas and leukaemias.

22. Compounds of the general formula (I), as defined previously, and/or their physiologically compatible salts, hydrates, isomers, more especially stereoisomers, tautomers and constitutional isomers, derivatives, prodrugs and metabolites for inhibiting topoisomerases and/or telomerases.

23. Compounds of the general formula (I), as defined previously; and/or their physiologically compatible salts, hydrates, isomers, more especially stereoisomers, tautomers and constitutional isomers, derivatives, prodrugs and metabolites, as regulators within the MAP kinasis signal transduction.

## Revendications

1. Utilisation de bicyclo[3.3.1]nonan-2,4,9-triones substituées de la formule générale 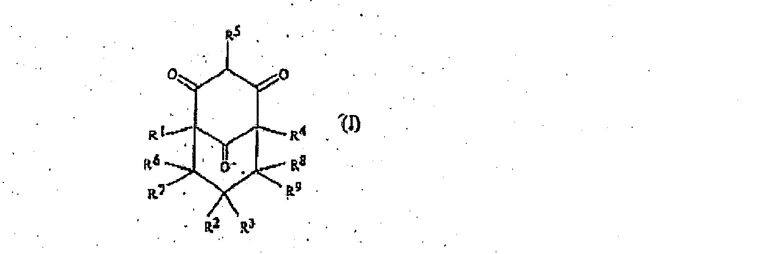 où
• R¹, R², R⁴ et R⁵ sont identiques ou différents l'un de l'autre, représentent indépendamment l'un de l'autre l'un des groupes suivants : 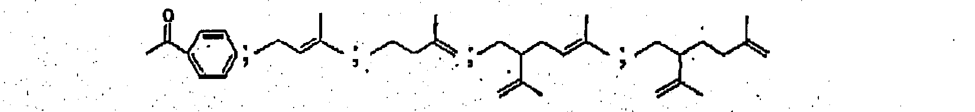 toutefois dans la mesure où, lorsque l'un des restes R¹ ou R² ou R4 ou R⁵ représente un groupe benzoyle, aucun autre de ces restes ne représente un groupe benzoyle;
• R³ représente un atome d'hydrogène ;
• R⁶ et R⁷ représentent tous deux, chacun, un groupe méthyle ou toutefois aussi, chacun, un atome d'hydrogène et R⁸ et R⁹ représentent, chacun un groupe méthyle ou toutefois aussi, chacun, un atome d'hydrogène,
toutefois dans la mesure où, lorsque R⁶ et R⁷ représentent des groupes méthyle, R⁸ et R⁹ sont des atomes d'hydrogène et que, lorsque R⁸ et R⁹ représentent des groupes méthyle, R⁶ et R⁷ sont des atomes d'hydrogène ;
et de leurs sels, hydrates, isomères, en particulier stéréoisomères, tautomères et isomères de constitution compatibles physiologiquement pour la fabrication de médicaments pour le traitement prophylactique et/ou thérapeutique de tumeurs et/ou cancers.

2. Utilisation selon la revendication 1, **caractérisée par le fait que** les liaisons de la formule (I) sont sélectionnées parmi le groupe des liaisons suivantes :

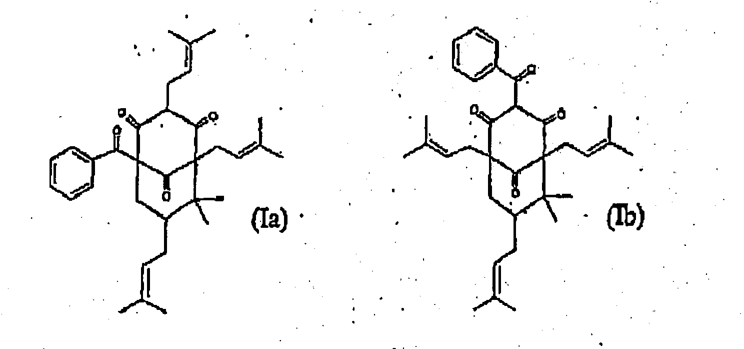

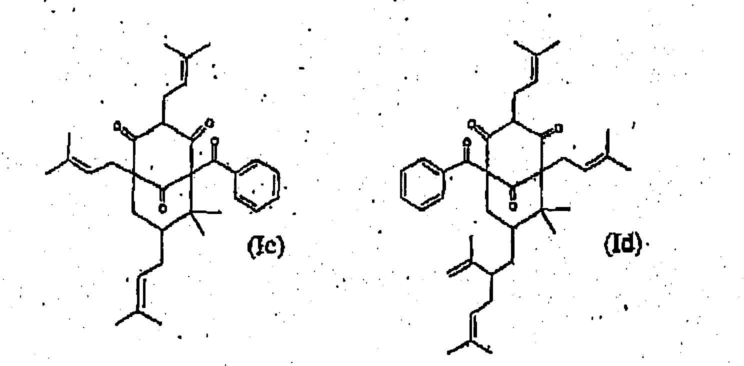

3. Utilisation selon la revendication 1 ou 2, **caractérisée par le fait que** la liaison de la formule (I) est là liaison suivante de la formule (Ic) :

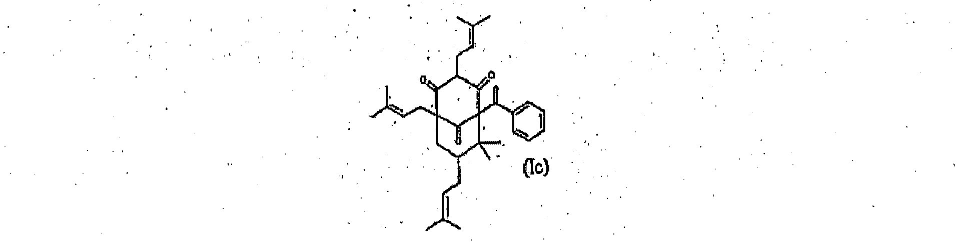

4. Utilisation selon la revendication 3, **caractérisée par le fait que** la liaison de la formule (Ic) présente la configuration suivante :

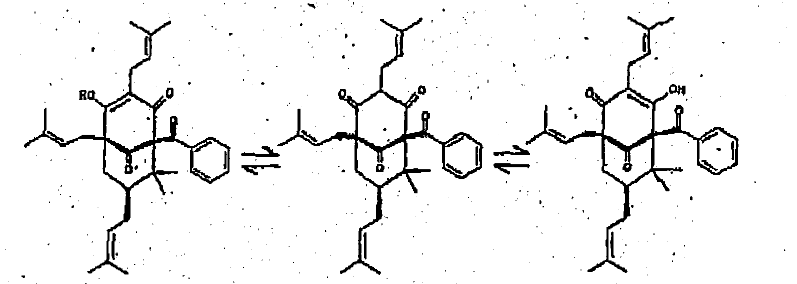

5. Utilisation selon l'une des revendications 1 à 4 pour le traitement prophylactique et/ou thérapeutique de tumeurs et/ou cancers, en particulier de tumeurs primaires et de métastases, et/ou de stades précancéreux.

6. Utilisation selon l'une des revendications 1 à 5 pour le traitement prophylactique et/ou thérapeutique de tumeurs bénignes et malignes.

7. Utilisation selon l'une des revendications 1 à 6 pour le traitement prophylactique et/ou thérapeutique du cancer de l'intestin (carcinomes du colon), du cancer du sein (carcinomes du sein), du cancer des ovaires, du cancer de l'utérus, du cancer des poumons, du cancer de l'estomac, du cancer du foie, des carcinomes du pancréas, du cancer des reins, du cancer de la vessie, du cancer de la prostate, du cancer des testicules, du cancer des os, du cancer de la peau, des sarcomes de Kaposi, des tumeurs du cerveau, des myosarcomes, des neuroblastomes, des lymphomes et des leucémies.

8. Utilisation selon l'une des revendications 1 à 7, **caractérisée par le fait que** les liaisons de la formulé générale (I) sont appliquées de manière systémique et/ou topique.

9. Utilisation selon l'une des revendications 1 à 8, **caractérisée par le fait que** les liaisons de la formule générale (I) freinent et/ou arrêtent la croissance et la division de cellules tumorales et/ou cancéreuses.

10. Utilisation selon l'une des revendications 1 à 9, **caractérisée par le fait que** les liaisons de la formule générale (I) induisent une destruction des DANN des cellules tumorales et/ou cancéreuses.

11. Utilisation de liaisons de la formule générale (I), telles que définies précédemment, et/ou de leurs sels, hydrates, isomères, en particulier stéréoïsomères, tautomères et isomères de constitution, dérivés, promédicaments et métabolites compatibles physiologiquement pour la fabrication d'inhibiteurs de topoïsomérase et/ou d'inhibiteurs de télomérase.

12. Utilisation selon l'une des revendications 1 à 11, **caractérisée par le fait que** les liaisons de la formule générale (I), telles que définies précédemment, et/ou leurs sels, hydrates, isomères, en particulier stéréoïsomères, tautomères et isomères de constitution, dérivés, promédicaments et métabolites compatibles physiologiquement sont utilisés en combinaison avec au moins un autre produit chimiothérapeutique pour la fabrication d'un médicament, en particulier des inhibiteurs de protéine kinase, de préférence des inhibiteurs de MAP kinase.

13. Cytostatique, contenant au moins une liaison de la formule générale (I), telle que définie précédemment, ét/ou ses sels, hydrates, isomères, en particulier stéréoïsomères, tautomères et isomères de constitution, dérivés, promédicaments et métabolites compatibles physiologiquement ensemble avec un porteur ou excipient compatible pharmaceutiquement, sensiblement non toxique.

14. Cytostatique, contenant l'au moins une liaison de la formule générale (I), telle que définie précédemment, et/ou ses sels, hydrates, isomères, en particulier stéréoïsomères, tautomères et isomères de constitution, dérivés, promédicaments et métabolites compatibles physiologiquement en des quantités thérapeutiquement actives.

15. Composition pharmaceutique ou médicament selon la revendication 13 ou 14, pour application systémique et/ou topique.

16. Combinaison pharmaceutique, en particulier combinaison cytostatique, comprenant
(A) au moins une liaison de la formule générale (I), telle que définie précédemment, et/ou ses sels, hydrates, isomères, en particulier stéréoïsomères, tautomères et isomères de constitution, dérivés, promédicaments et métabolites compatibles physiologiquement ; et
(B) au moins un autre produit chimiothérapeutique, en particulier un inhibiteur de protéine kinase, de préférence un inhibiteur de MAP kinase.

17. Combinaison pharmaceutique selon la revendication 16, **caractérisée par le fait que** les composants (A) et (B) sont présents soit sous forme d'unité fonctionnelle, en particulier sous forme de mélange ou de composition, soit aussi (spatialement) séparément l'un de l'autre.

18. Combinaison pharmaceutique selon la revendication 16 ou 17, les composants (A) et (B) pouvant être utilisés ou appliqués soit en même temps, soit aussi par étapes dans le temps.

19. Combinaison pharmaceutique selon l'une des revendications 16 à 18, contenant les composants (A) et (B) chaque fois en des quantités thérapeutiquement actives.

20. Combinaison pharmaceutique selon l'une des revendications 16 à 19, pour application systémique et/ou topique.

21. Liaisons de la formule générale (I), telles que définies précédemment, et/ou leurs sels, hydrates, isomères, en particulier stéréoïsomères, tautomères et isomères de constitution, dérivés, promédicaments et métabolites compatibles physiologiquement pour le traitement prophylactique et/ou thérapeutique de cancers, en particulier de tumeurs primaires et de métastases, et/ou de stades précancéreux, en particulier du cancer de l'intestin (carcinomes du colon), du cancer du sein (carcinomes du sein), du cancer des ovaires, du cancer de l'utérus, du cancer des poumons, du cancer de l'estomac, du cancer du foie, des carcinomes du pancréas, du cancer des reins, du cancer de la vessie, du cancer de la prostate, du cancer des testicules, du cancer des os, du cancer de la peau, des sarcomes de Kaposi, des tumeurs du cerveau, des myosarcomes, des neuroblastomes, des lymphomes et des leucémies.

22. Liaisons de la formule générale (I), telles que définies précédemment, et/ou leurs sels, hydrates, isomères, en particulier stéréoïsomères, tautomères et isomères de constitution, dérivés, promédicaments et métabolites compatibles physiologiquement pour l'inhibition de topoïsomérases et/ou de télomérases.

23. Liaisons de la formule générale (I), telles que définies précédemment, et/ou leurs sels, hydrates, isomères, en particulier stéréoïsomères, tautomères et isomères de constitution, dérivés, promédicaments et métabolites compatibles physiologiquement en tant que régulateurs dans la transduction de signaux de MAP kinase.
